# EUROPEAN PATENT SPECIFICATION

(11) **EP 1 158 977 B1**
(45) Date of publication and mention of the grant of the patent: **12.10.2005**
(21) Application number: 00916179.5
(22) Date of filing: 09.03.2000
(51) Int. Cl.: A61K 31/40, A61K 31/635

(54) **COMPOSITION FOR ADMINISTERING A CYCLOOXYGENASE-2 INHIBITOR TO ANIMALS**
ZUSAMMENSETZUNGEN ZUR VERABREICHUNG EINES CYCLOOXYGENASE-2-HEMMERS AN TIERE
COMPOSITION SE RAPPORTANT A L'ADMINISTRATION D'UN INHIBITEUR DE LA CYCLO-OXYGENASE-2 AUX ANIMAUX

(30) Priority: 10.03.1999 US 123644 P
(43) Date of publication of application: 05.12.2001
(73) Proprietor: G.D. Searle LLC, St. Louis, Missouri 63141 (US)
(72) Inventor: KEANE, J., Timothy, Clayton, MO 63105 (US); SCHUH, Joseph, R., St. Louis, MO 63146 (US); SCRIVNER, Alan, L., Wildwood, MO 63038 (US)
(74) Representative: Albrecht, Thomas
(86) International application number: PCT/US2000/006097
(87) International publication number: WO 2000/053149

(56) References cited:
- WO-A-98/17292
- WO-A-98/22101
- WO-A-98/50033
- WO-A1-96/38418
- WO-A1-98/50033
- US-A- 5 756 529
- CANCER RESEARCH, vol. 58, 1998, pages 409-412, XP001145866
- VASSEUR ET AL.: 'Randomized, controlled trial of the efficacy of carprofen, a nonsteroidal anti-inflammatory drug, in the treatment of osteoarthritis in dogs' JAVMA, vol. 206, no. 6, 15 March 1995, pages 807 - 811, XP002931074
- LASCELLES ET AL.: 'Carprofen as an analgesic for postoperative pain in cats: Dose titration and assessment of efficacy in comparison to pethidine hydrochloride' J. SMALL ANIM. PRACT., vol. 36, no. 12, 1995, pages 535 - 541, XP002931097
- FOX ET AL.: 'Use of carprofen for the treatment of pain and inflammation in dogs' JAVMA, vol. 210, no. 10, 15 May 1997, pages 1493 - 1498, XP002931075
- RICKETTS ET AL.: 'Evaluation of selective inhibition of canine cyclooxygenase 1 and 2 by carprofen and other nonsteroidal anti-inflammatory drugs' AM. J. VET. RES., vol. 59, no. 11, November 1998, pages 1441 - 1446, XP002931076
- KHAN ET AL.: 'Gastrointestinal and renal safety of a specific cox-2 inhibitor versus NSAIDs in dogs' VET PATHOL., vol. 34, no. 5, 1997, page 509, ABSTRACT NO. 150, XP002931051
- HAVEN ET AL.: 'In Vitro and Ex Vivo whole blood evidence of selective cyclooxygenase-2 inhibition by rimadyl (carprofen)' J. VET. INTERN. MED., vol. 12, no. 3, May 1998 - June 1998, page 243, ABSTRACT NO. 182, XP002931052

## Description

### FIELD OF THE INVENTION

The present invention relates to articles of manufacture comprising compositions for treatment or prophylaxis of a cyclooxygenase-2 mediated condition or disorder in a non-human animal, comprising at least one cyclooxygenase-2 inhibitor.

### BACKGROUND OF THE INVENTION

There are few drugs that can be successfully used in veterinary medicine for treatment or prophylaxis of inflammation and inflammation-related conditions and disorders. Such limited options as are available are listed in standard reference works, for example K. Bennett, Compendium of Veterinary Products (Second Edition, 1993), and The Merck Veterinary Manual, pp. 1504-1509 (Seventh Edition, 1991).

Isakson *et al.,* U.S. Patent No. 5,756,529, describe use of a class of substituted pyrazolyl benzenesulfonamides in treatment of inflammation and inflammation-related disorders in companion animals.

Vasseur *et al.,* J. Am. Vet. Med. Assoc., 206(6), 807-81 (1995), describe use of carprofen for the treatment of osteoarthritis in dogs. Carprofen is a non-steroidal anti-inflammatory drug (NSAID) that is available in chewable tablets said to be palatable to dogs. See product literature on Rimadyl® chewable tablets of Pfizer.

Lundy *et al.,* WO 98/50033, describe use of carprofen for treatment of pain and inflammation in dogs.

Knapp *et al*., J. Vet. Int. Med., 8, 273 (1994) describe use of piroxicam for treatment of carcinomas in dogs.

Not only are the available veterinary medicines for treatment or prophylaxis of inflammation and inflammation-related conditions and disorders significantly limited, those few that are available are difficult to administer to an animal subject. Where a therapeutic agent is administered by the owner or keeper of the animal, as is frequently the case with, for example, companion animals, working animals, farm livestock and breeding stock, the preferred route of administration typically is oral.

However, administration of medication in conventional oral dosage forms to animals can be extremely difficult, in part because most animals, whether through instinctive, learned or reflex behavior, tend to reject non-food items from the mouth rather than swallowing such items. For conventional unit dosage forms such as pills, including capsules, tablets and the like, the animal's mouth typically must be held open while the pill is inserted into the back of the throat. The animal's mouth is then held closed to encourage swallowing. It often takes two people to administer the medication and can result in injury or distress to the person administering the medication, to the animal or to both. Animal owners and keepers have tried to conceal a pill and/or mask its taste by wrapping the pill in a food product such as cheese or meat and then feeding the food product to the animal, but the pill often escapes its wrapping or is otherwise detected by the animal and expelled from the animal's mouth.

Several patents have proposed overcoming this problem by using edible materials in which a pill can be hidden with less risk of detection by a subject animal. These patents do not identify specific therapeutic agents that can be administered using the invention, but rather apply to the administration of conventional dosage forms of therapeutic agents generally.

Harold, U.S. Patent No. 4,857,333, describes a food treat for animals, such as a bone-shaped rigid dry dog food treat or a soft-formed rounded dog treat, that contains an interior pocket sized to conceal and retain a pill for treatment or prevention of animal diseases.

Durand *et al.,* U.S. Patent No. 5,853,757, describe a carrier for animal medication. The carrier is formed from a soft edible material and has an interior chamber into which medication can be placed. The carrier masks the scent of the medication and further comprises a lubricant to assist with the consumption of the carrier.

Baumgardner, U.S. Patent No. 5,792,470, describes an edible container for administering medication to an animal. The container comprises a length of a swaged tubular member that the animal may consume. This tubular member is constructed from an edible material and conceals the medication within the member.

Languet *et al.,* U.S. Patent No. 5,747,063, describe an envelope comprising, in admixture, one or more "craved for materials" and one or more materials agglomerating the "craved for materials". The envelope is hollow and can be used to conceal an oral medication when the envelope is administered to an animal.

Several other patents and applications for patent have proposed oral administration of certain compounds to animals by incorporating such compounds into an animal feed. This approach, however, is limited by, for example, the chemical and physical stability of the compound incorporated into the feed, the interaction of the compound with other ingredients of the feed, and the processing steps to which the compound is subjected. Few of these patents and applications for patent have suggested incorporation of a systemic therapeutic agent into an animal feed, and none has suggested that a non-steroidal anti-inflammatory drug (NSAID) or, more particularly, a selective cyclooxygenase-2 inhibitory drug, should or could be incorporated into an animal feed.

Garnett, U.S. Patent No. 5,759,537, describes a kit comprising a bacterial strain and a substrate. The bacterial strain and substrate are added to an animal feed wherein the bacterial strain produces an enzyme that converts the substrate into a lysophospholipid having growth promoting properties when fed to animals.

Richar, U.S. Patent No. 5,405,836, describes the preparation of a farinaceous-based baked or cooked pet food comprising a topically applied water-soluble zinc salt that controls malodorous breath when the pet food is chewed by a pet.

Edwards, U.S. Patent No. 5,316,770, describes a feed composition containing a hydroxylated vitamin D₃ derivative that can be fed to animals, particularly poultry, for enhancement of phytate phosphorus utilization and treatment and prevention of tibial dyschondroplasia.

Kealy *et al.,* U.S. Patent No. 4,772,476, describe a method for reducing the severity of hip dysplasia in animals wherein the animals are fed a nutritionally balanced composition in which the dietary electrolyte balance in the composition is maintained at a level that is not greater than about 20 milliequivalents/100 g.

Berschneider *et al.,* German Democratic Republic Patent DD-88,879, describe preparation of an animal feed for treating pathogen-caused gastrointestinal diseases in animals wherein the feed comprises antibiotics, sulfonamides and chemotherapeutic agents.

Weil, German Democratic Republic Patent DD-247,843, describes a method for production of a specific diluent for physiologically active ingredients administered to animals. The diluent comprises a basic magnesium compound, dehydrated sodium acetate and organic dicarboxylic acid. The active ingredient is mixed with the diluent and administered orally to the animal, such as in combination with food or beverage.

Dugger *et al.,* WO 98/47392, describe an animal food product containing at least one edible component (which may include animal nutritional materials, animal immune system stimulants, animal appetite suppressants, animal color enhancers or animal therapeutic agents) and an edible gel carrier matrix.

Fuller, U.S. Patent No. 4,294,857, describes a dog food composition prepared by incorporating 3,7-dimethyl-1,6-octadien-3-ol into the composition in an amount of about 0.0001 % to about 0.001 % to improve palatability of the composition.

Islam, U.S. Patent No. 4,346,118, describes incorporation of dialkyl esters of fumaric acid in an animal feed as an anti-fungal agent to resist microbial attack and spoilage of the feed.

Kawamori *et al.,* Cancer Research 58, 409-412. (1998) describe a study of chemopreventive activity of the cyclooxygenase-2 inhibitor Celecoxib in which rats having a body weight of less than 500 g were fed a standard diet (modified AIN-76A) with which Celecoxib was mixed.

There remains a need for an inexpensive, easy to administer oral composition that delivers a medicine to a non-human animal for treatment or prophylaxis of a cyclooxygenase-2 mediated condition or disorder, particularly inflammation or an inflammation-related condition or disorder, without the conventional problems attendant upon administration of pills such as tablets, capsules and the like. In particular, there remains a need for a food composition or article of manufacture that delivers such a medicine to a non-human animal in dose units that are readily, conveniently and accurately meterable by eye.

### SUMMARY OF THE INVENTION

The present invention provides an article of manufacture comprising a shaped composition having two substantially planar ends, an elongate dimension substantially orthogonal to the ends and a substantially uniform cross-sectional area, the shaped composition comprising a food material having substantially homogeneously dispersed therein a selective cyclooxygenase-2 inhibitor, the shaped composition being packaged in a cuttable wrapping material having printed thereon marks at equal spacing along the elongate dimension, these marks corresponding to increments of dosage amount of the cyclooxygenase-2 inhibitor contained in portions of the shaped composition defined by the marks.

In yet another particular embodiment, the present invention also provides an article of manufacture comprising a shaped composition that comprises a brittle food material having substantially homogeneously dispersed therein a selective cyclooxygenase-2 inhibitor, the shaped composition having means for providing linear zones of reduced mechanical strength permitting breakage into substantially evenly sized portions each containing a metered dosage amount of the cyclooxygenase-2 inhibitor.

Other features of the invention will be in part apparent and in part pointed out hereinafter.

### BRIEF DESCRIPTION OF THE DRAWINGS

Certain aspects of the invention will be better understood from the description that follows, given by way of non-limiting example, with reference to the accompanying drawings, in which:
Fig. 1 shows a perspective view of an article of manufacture of one embodiment of the invention.
Fig. 2 shows a plan view of an article of manufacture of an embodiment of the invention related to the embodiment shown in Fig. 1.
Fig. 3 shows a perspective view of an article of manufacture of another embodiment of the invention.
Fig. 4 shows a perspective view of an article of manufacture of another embodiment of the invention.

### DESCRIPTION OF ILLUSTRATIVE EMBODIMENTS

Unique compositions have been discovered that may be used to administer a desired amount of a cyclooxygenase-2 inhibitor to an animal with, among other benefits, improved ease and dosage regulation. Fundamentally, the composition is a food composition, such as a human food composition or an animal food composition, comprising at least one cyclooxygenase-2 inhibitor. The composition provides a cyclooxygenase-2 inhibitor to an animal at a dosage that is sufficient to provide prolonged inhibition of cyclooxygenase-2 and thus confer the desired therapeutic benefit while maintaining a safe clearance time for the inhibitor.

In particular, the compositions of the present invention:
(1) are easier to administer than conventional pharmaceutical compositions such as pills, tablets and the like;
(2) contain an evenly distributed and constant amount of cyclooxygenase-2 inhibitor per unit volume of the food composition;
(3) allow the animal owner to make a relatively precise measurement of the dosage of the cyclooxygenase-2 inhibitor administered to and consumed by the animal. The dosage administered to the animal is proportional to the volume of a ration of the food composition consumed by the animal. Where the animal owner knows the weight of the animal and the specific dosage (mg inhibitor/kg animal body weight) of cyclooxygenase-2 inhibitor desired, the owner can easily and accurately administer that dosage by feeding the animal a corresponding ration of the composition; and/or
(4) have an extended shelf-life making them appropriate for retail sale in the same manner as non-medicated animal food products.

### Utiliy of Compositions

The compositions of the present invention would be useful for, but not limited to, the treatment or prophylaxis of inflammation and/or inflammation-associated conditions and disorders in an animal, and for treatment or prophylaxis of other cyclooxygenase-2 mediated conditions and disorders, such as, use as an analgesic in the treatment of pain, or as an antipyretic for the treatment of fever. For example, compositions of the invention would be useful to treat inflammation of the musculoskeletal system, including chronic inflammation of hard and soft tissues, joint disease and traumatic injury. The compositions would be useful to treat arthritis, including but not limited to, rheumatoid arthritis, gouty arthritis, and osteoarthritis, myositis, and tendonitis. Such compositions of the invention would be useful in the treatment of equine colic, mastitis, peritonitis, and skin-related conditions such as bums and dermatitis. Compositions of the invention also would be useful to treat gastrointestinal conditions such as gastritis, ulcerative colitis, viral and bacterial infections of the gastrointestinal tract, and for the prevention of cancer, including colorectal cancer. Compositions of the invention would be useful in treating inflammation in such diseases as vascular diseases, gingivitis, hypersensitivity, conjunctivitis, and other eye inflammation, swelling occurring after injury or surgery, myocardial ischemia, and the like. The compositions also would be useful for cognitive enhancement and in treating cognitive dementia. The compositions are useful as anti-inflammatory agents, such as for the treatment of arthritis, with the additional benefit of having significantly less harmful side effects.

Preferred uses for the compositions of the present invention, however, are for the treatment or prophylaxis of inflammation and inflammation-related conditions and disorders in animals, particularly rheumatoid arthritis, osteoarthritis, cognitive dementia, cancer and pain management generally.

In one embodiment, for example, a composition of the present invention is administered to a non-human animal that is susceptible to or is suffering from inflammation or an inflammation-related disorder.

### Administration To Non-Human Subjects

These compositions are intended for non-human use. They are useful for the veterinary treatment of animals, particularly companion animals, zoo animals, exotic animals and farm animals, including mammals, rodents, avians, and the like. Preferred companion animals include, but are not limited to, dogs, cats, horses, rabbits, guinea pigs and ferrets. Preferred farm animals include, but are not limited to, cattle, swine, sheep, goats and poultry. More preferably, the compositions of the present invention are administered to the group consisting of dogs, cats and horses. Still more preferably, the compositions of the present invention are administered to the group consisting of dogs and horses.

The compositions of the present invention are administered to non-human animals having a body weight greater than about 1 kg. The animal preferably has a body weight between about 1 kg to about 5000 kg, more preferably between about 1.5 kg to about 3000 kg, and still more preferably between about 2 kg to about 2000 kg. In one embodiment, for example, the animal has a body weight between about 2 kg to about 70 kg. Such animals typically include, but are not limited to, dogs. In another embodiment, for example, the animal has a body weight between about 50 kg to about 1500 kg. Such animals typically include, but are not limited to, horses.

### Cyclooxygenase-2 Inhibitors

The term "cyclooxygenase-2 inhibitor" means any pharmaceutically acceptable compound or combination of compounds, including salts, tautomers and prodrugs of such compound or compounds, that inhibits the enzyme cyclooxygenase-2 in the arachidonic acid/prostaglandin pathway and are selected from the compounds corresponding to the structural formula: 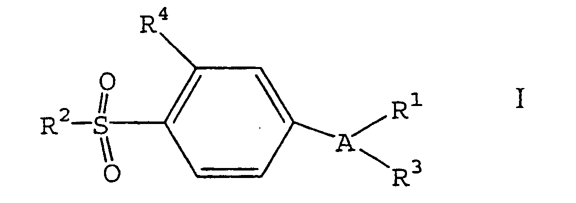 wherein A is a 5- or 6-member ring substituent selected from partially unsaturated or unsaturated heterocyclo and carbocyclic rings;
wherein R¹ is cyclohexyl or phenyl optionally substituted with one, two or three radicals selected from C₁₋₂ alkyl, C₁₋₂ haloalkyl, cyano, carboxyl, C₁₋₂ alkoxycarbonyl, hydroxyl, C₁₋₂ hydroxyalkyl, C₁₋₂ haloalkoxy, amino, C₁₋₂ alkylamino, phenylamino, nitro, C₁₋₂ alkoxy-C₁₋₂-alkyl, C₁₋₂ alkylsulfinyl, halo, C₁₋₂ alkoxy and C₁₋₂ alkylthio;
wherein R² is methyl or amino;
wherein R³ is a radical selected from halo, C₁₋₂ alkyl, alkenyl, alkynyl, oxo, cyano, carboxyl, cyanoalkyl, heterocyclyloxy, C₁₋₂ akyloxy, alkylthio, alkylcarbonyl, cycloalkyl, phenyl, C₁₋₂ haloalkyl, heterocyclo, cycloalkenyl, phenylalkyl, heterocyclylalkyl, alkylthioalkyl, C₁₋₂ hydroxyalkyl, alkoxycarbonyl, phenylcarbonyl, phenylalkylcarbonyl, phenylalkenyl, alkoxyalkyl, phenylylthioalkyl, phenylyloxyalkyl, phenylalkylthioalkyl, phenylalkoxyalkyl, alkoxyphenylalkoxyalkyl, alkoxycarbonylalkyl, aminocarbonyl, aminocarbonylalkyl, alkylaminocarbonyl, N-phenylaminocarbonyl, N-alkyl-N-phenylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-phenylaminoalkyl, N-phenylalkylaminoalkyl, N-alkyl-N-phenylalkylaminoalkyl, N-alkyl-N-phenylaminoalkyl, phenyloxy, phenylalkoxy, phenylthio, phenylalkylthio, alkylsulfinyl, alkylsulfonyl, aminosulfonyl, alkylaminosulfonyl, N-phenylaminosulfonyl, phenylsulfonyl and N-alkyl-N-phenylaminosulfonyl; and
wherein R⁴ is hydrido or fluoro;
or a pharmaceutically-acceptable salt thereof.

A class of cyclooxygenase-2 inhibitors of particular interest consists of those compounds of Formula I:
wherein A is a 5- or 6-member ring substituent selected from partially unsaturated or unsaturated heterocyclo and carbocyclic rings selected from the group consisting of thienyl, oxazolyl, furyl, pyrrolyl, thiazolyl, imidazolyl, benzofuryl, indenyl, benzothienyl, isoxazolyl, pyrazolyl, cyclopentenyl, cyclopentadienyl, benzindazolyl, benzopyranopyrazolyl, phenyl, and pyridyl;
wherein R¹ is cyclohexyl or phenyl optionally substituted with one, two or three radicals selected from C₁₋₂ alkyl, halo, and C₁₋₂ alkoxy;
wherein R² is methyl or amino;
wherein R³ is a radical selected from halo, C₁₋₂ alkyl, oxo, cyano, carboxyl, C₁₋₂ alkyloxy, phenyl, C₁₋₂ haloalkyl, and C₁₋₂ hydroxyalkyl; and
wherein R⁴ is hydrido or fluoro;
or a pharmaceutically-acceptable salt thereof.

Preferred are cyclooxygenase-2 inhibitors selected from the group consisting of:
(1) the compound 4-[5-(4-methylphenyl)-3-(trifluoromethyl)-1H-pyrazole-1-yl]benzenesulfonamide (also referred to herein as Celecoxib) which has the structure: 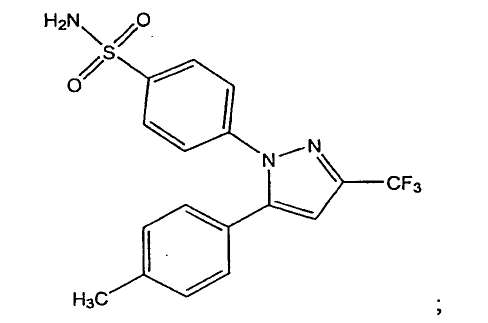
(2) the compound 4-[5-((3-fluoro-4-methoxy)phenyl)-3-(difluoromethyl)-1H-pyrazole-1-yl]benzenesulfonamide (also referred to herein as Deracoxib) which has the structure: 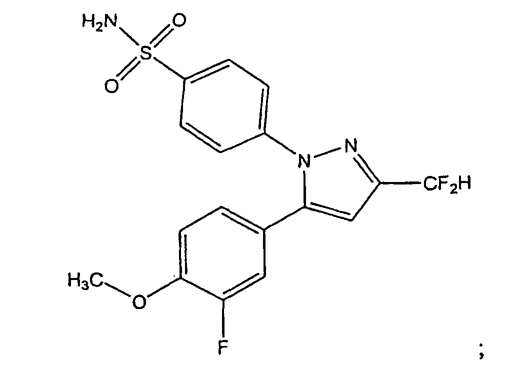
(3) the compound 4-[5-(4-chlorophenyl)-3-(trifluoromethyl)-1H-pyrazole-1-yl]benzenesulfonamide which has the structure: 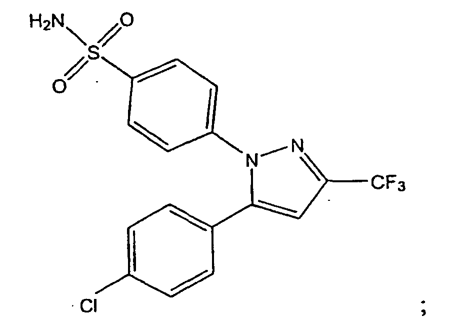
(4) the compound that has the structure: 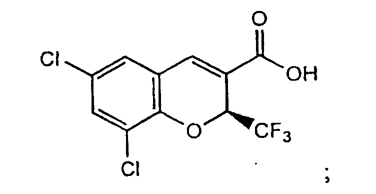
(5) the compound 4-[4-(methylsulfonyl)phenyl]-3-phenylfuran-2(5H)-one (also referred to herein as Rofecoxib) which has the structure: 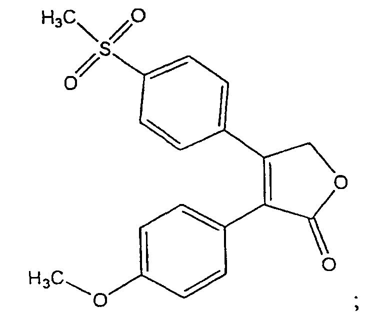 and
(6) the compound 4-[5-methyl-3-phenylisoxazol-4-yl]benzenesulfonamide, (also referred to herein as Valdecoxib) which has the structure:

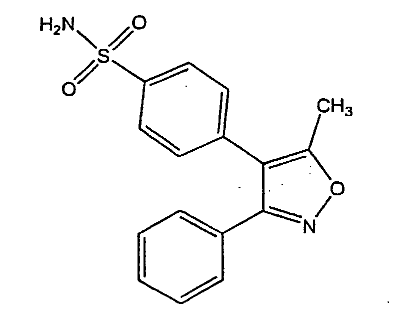

In one illustrative embodiment, the cyclooxygenase-2 inhibitor is selected from the group consisting of Celecoxib, Deracoxib, Valdecoxib and Rofecoxib.

In another embodiment, the cyclooxygenase-2 inhibitor is selected from the group consisting of Deracoxib, Valdecoxib and Rofecoxib.

In still another embodiment, the cyclooxygenase-2 inhibitor is Deracoxib.

In still another embodiment, the cyclooxygenase-2 inhibitor is Valdecoxib.

In still another embodiment, the cyclooxygenase-2 inhibitor is Rofecoxib.

In still another embodiment, the cyclooxygenase-2 inhibitor is Celecoxib.

While the selection of the cyclooxygenase-2 inhibitor is not narrowly critical, certain cyclooxygenase-2 inhibitors may be preferable in view of the physical and chemical properties of the inhibitor, the food composition selected, and the specific process selected for the preparation of the food composition. Such considerations will be apparent to one skilled in the art. For example, where the process for the preparation of the food composition requires the use of elevated temperatures, it is preferable to select a cyclooxygenase-2 inhibitor that does not degrade at such temperatures.

The use of a selective cyclooxygenase-2 inhibitor that minimizes or avoids the detrimental or unwanted side-effects resulting from cyclooxygenase-1 inhibition generally is preferred. The term "selective cyclooxygenase-2 inhibitor" generally means a cyclooxygenase-2 inhibitor that has selectivity ratio of cyclooxygenase-2 inhibition over cyclooxygenase-1 inhibition greater than 1. Preferably, the inhibitor has a cyclooxygenase-2 IC₅₀ of less than about 0.2 µM, and also has a selectivity ratio of cyclooxygenase-2 inhibition over cyclooxygenase-1 inhibition of at least 50, and more preferably of at least 100. Even more preferably, the inhibitor has a has a cyclooxygenase-1 IC₅₀ of greater than about 1 µM, and more preferably of greater than about 10 µM. Such preferred selectivity may indicate an ability to reduce the incidence of common non-steroidal anti-inflammatory drug-induced side effects.

### Cyclooxygenase-2 Inhibitor Dosages

The daily amount of cyclooxygenase-2 inhibitor administered to the animal preferably is between 0.1 mg/kg animal body weight to 15 mg/kg animal body weight, more preferably between 0.5 mg/kg animal body weight to 10 mg/kg animal body weight, and still more preferably between 1 mg/kg animal body weight to 5 mg/kg animal body weight. The animal food compositions described herein, such as, for example, pet foods, comprise an amount of the cyclooxygenase-2 inhibitor that is proportional to the volume of a ration of the composition. Therefore, the daily ration of animal food composition, and thus the dosage of inhibitor, to be administered can be easily determined based on the weight of the animal and the desired or recommended amount of cyclooxygenase-2 inhibitor/animal body weight.

Because the amount of cyclooxygenase-2 inhibitor administered typically is relatively small in comparison to the amount of the food composition containing the cyclooxygenase-2 inhibitor, conventional daily rations of the animal feed composition can be maintained. The total daily amount of the composition fed to the animal may be in the form of one or more individual servings, such as, but not limited to, one to four servings. Preferably, the total daily amount of the composition fed to the animal is in the form or one or two individual servings.

Additionally, the food compositions of the present invention may be the sole source of food for the animal or they may be combined with other food sources that do not contain a cyclooxygenase-2 inhibitor. In one embodiment, for example, the food composition is the sole food source for the animal. This allows the animal owner to more readily ensure the complete consumption of the inhibitor by the animal and monitor such consumption.

The dosage regimen, and thus the amount of food composition that is administered, for treating the intended condition or disorder will depend on a variety of factors, including the age, weight, sex and medical condition of the animal, the severity of the condition or disorder, the route and frequency of administration, and thus may vary.

### Carrier Materials

The compositions of the present invention may comprise an edible carrier material, such as an animal food composition, comprising a therapeutically effective amount of unformulated cyclooxygenase-2 inhibitor. In this embodiment, the edible carrier material acts as the sole carrier material for the cyclooxygenase-2 inhibitor. In many applications, however, it is desirable to prepare an initial inhibitor composition comprising a desired amount of the cyclooxygenase-2 inhibitor in combination with one or more additional pharmaceutically-acceptable carrier materials appropriate for oral administration. The inhibitor composition is then combined with the edible carrier material. The compositions of the present invention, therefore, may comprise the cyclooxygenase-2 inhibitor in a desired amount admixed with, not only the edible carrier material, but also one or more carrier materials selected from the group consisting of pharmaceutically acceptable diluents, disintegrants, binding agents and adhesives, wetting agents, lubricants, anti-adherent agents and/or other carrier materials.

The term "edible carrier material" as used herein means an organic material that can be digested or passed through the digestive system of an animal without any toxic or noxious effects to the same animal. These edible carrier materials can exist as either a solid or liquid at room temperature, preferably liquid. Preferred carrier materials will have properties that allow for solubility of cyclooxygenase-2 inhibitors therein for easy homogeneous dispersion of cyclooxygenase-2 inhibitors therein.

### Diluents

Although the animal food composition itself can serve as a diluent, the compositions of the present invention optionally may comprise one or more additional pharmaceutically-acceptable diluents as a carrier material. Because the amount of cyclooxygenase-2 inhibitor required per kilogram of animal food composition is relatively small, it may be desirable to use a diluent to increase the bulk, and therefore the ease of handling, of the cyclooxygenase-2 inhibitor composition prior to addition to the animal food composition. Suitable diluents may include, either individually or in combination, such diluents as lactose USP; lactose USP, anhydrous; lactose USP, spray dried; starch USP; directly compressible starch; mannitol USP; sorbitol; dextrose monohydrate; microcrystalline cellulose NF; dibasic calcium phosphate dihydrate NF; sucrose-based diluents; confectioner's sugar; monobasic calcium sulfate monohydrate; calcium sulfate dihydrate NF; calcium lactate trihydrate granular NF; dextrates, NF (e.g., Emdex); Celutab; dextrose (e.g., Cerelose); inositol; hydrolyzed cereal solids such as the Maltrons and Mor-Rex; amylose; Rexcel; powdered cellulose (e.g., Elcema); calcium carbonate; glycine; bentonite; polyvinylpyrrolidone; and the like. The inhibitor composition prior to addition to the animal food composition may comprise, for example, one or more diluents in the range of about 5% to about 99%, preferably about 10% to about 85%, and more preferably about 20% to about 80%, of the total weight of the composition. The diluent or diluents selected preferably exhibit suitable flow properties. Lactose and microcrystalline cellulose, either individually or in combination, are representative diluents. Because the animal food composition itself serves as a carrier material, the addition of further diluents directly to the food composition generally is not necessary.

### Disintegrants

The inhibitor compositions of the present invention optionally may comprise one or more pharmaceutically-acceptable disintegrants as a carrier. Suitable disintegrants may include, either individually or in combination, such disintegrants as starches; sodium starch glycolate; clays (such as Veegum HV); celluloses (such as purified cellulose, methylcellulose and sodium carboxymethylcellulose, and carboxymethylcellulose); alginates; pregelatinized corn starches (such as National 1551 and National 1550); Crospovidone, USP NF; gums (such as agar, guar, locust bean, Karaya, pectin, and tragacanth). The inhibitor composition prior to addition to the animal food composition may comprise, for example, one or more disintegrants in the range of about 0.2% to about 30%, preferably about 0.2% to about 10%, and more preferably about 0.2% to about 5%, of the total weight of the inhibitor composition. Croscarmellose sodium is a representative disintegrant, preferably in the range of about 0.2% to about 10%, more preferably in the range of about 0.2% to about 6%, and still more preferably in the range of about 0.2% to about 5%, by weight of the inhibitor composition. Additionally, disintegrants may be added directly to the animal food composition at any suitable step during the preparation of the animal food composition. The chewing action of the animal, however, generally causes a suitable disintegration of the animal food composition without the need for the addition of disintegrants to the animal feed composition.

### Binding Agents and Adhesives

The inhibitor compositions optionally may comprise one or more pharmaceutically-acceptable binding agents or adhesives as a carrier. Such binding agents and adhesives preferably impart sufficient cohesion to the powders to allow for normal processing such as sizing, lubrication, and packaging, but still allow the inhibitor composition to disintegrate and dissolve upon ingestion. Suitable binding agents and adhesives may include, either individually or in combination, such binding agents and adhesives as acacia; tragacanth; sucrose; gelatin; glucose; starch; cellulose materials such as, but not limited to, methylcellulose and sodium carboxymethylcellulose (e.g., Tylose); alginic acid and salts of alginic acid; magnesium aluminum silicate; polyethylene glycol; guar gum; polysaccharide acids; bentonites; polyvinylpyrrolidone; polymethacrylates; hydroxypropylmethylcellulose (HPMC); hydroxypropylcellulose (Klucel); ethylcellulose (Ethocel); pregelatinized starch (such as National 1511 and Starch 1500).

The inhibitor composition prior to addition to the animal food composition may comprise, for example, one or more binding agents and/or adhesives in the range of about 0.5% to about 25%, preferably about 0.75% to about 15%, and more preferably about 1% to about 10%, of the total weight of the composition. Polyvinylpyrrolidone is a representative binding agent used impart cohesive properties to the powder blend of the inhibitor composition. The inhibitor composition may comprise, for example, polyvinylpyrrolidone in a range of about 0.5% to about 10%, more preferably about 0.5% to about 7%, and still more preferably about 0.5% to about 5%.

It also may be desirable to add one or more binding or adhesive agents directly to the animal food composition to assist with the admixing of the cyclooxygenase-2 inhibitor with the food composition itself.

### Wetting Agents

Some cyclooxygenase-2 inhibitors are largely insoluble in aqueous solution. Accordingly, the inhibitor compositions of the present invention optionally may comprise one or more pharmaceutically-acceptable wetting agents as a carrier material. Such wetting agents can help to maintain the dispersion of the cyclooxygenase-2 inhibitor, particularly in high moisture animal food compositions, and can improve the relative bioavailability of the cyclooxygenase-2 inhibitor upon ingestion by the animal. Suitable wetting agents may include, either individually or in combination, such wetting agents as oleic acid; glyceryl monostearate; sorbitan monooleate; sorbitan monolaurate; triethanolamine oleate; polyoxyethylene sorbitan monooleate; polyoxyethylene sorbitan monolaurate; sodium oleate; and sodium lauryl sulfate. Wetting agents that are anionic surfactants are preferred.

The inhibitor composition prior to addition to the animal food composition may comprise, for example, one or more wetting agents in the range of about 0.25% to about 15%, preferably about 0.4% to about 10%, and more preferably about 0.5% to about 5%, of the total weight of the composition. Sodium lauryl sulfate is a representative wetting agent. The inhibitor compositions preferably comprise sodium lauryl sulfate as the wetting agent in the range of about 0.25% to about 7%, more preferably about 0.4% to about 6%, and still more preferably about 0.5 to about 5%.

### Lubricants

The inhibitor compositions may comprise one or more pharmaceutically-acceptable lubricants and/or glidants as a carrier material. Suitable lubricants and/or glidants may include, either individually or in combination, such lubricants and/or glidants as glyceryl behapate (Compritol 888); stearates (magnesium, calcium, sodium); stearic acid; hydrogenated vegetable oils (e.g., Sterotex); talc; waxes; Stearowet; boric acid; sodium benzoate and sodium acetate; sodium fumarate; sodium chloride; DL-Leucine; polyethylene glycols (e.g., Carbowax 4000 and Carbowax 6000); sodium oleate; sodium benzoate; sodium acetate; sodium lauryl sulfate; and magnesium lauryl sulfate.

The inhibitor composition prior to addition to the animal food composition may comprise, for example, one or more lubricants in the range of about 0.1 % to about 10%, preferably about 0.2% to about 8%, and more preferably about 0.25% to about 5%, of the total weight of the composition. Magnesium stearate is a representative lubricant used, for example, to reduce friction between the equipment and inhibitor composition during blending. It also may be desirable to add one or more lubricants directly to the animal food composition to assist with the admixing of the cyclooxygenase-2 inhibitor with the food composition itself.

Other carrier materials (such as anti-adherent agents, colorants, flavors, sweeteners and preservatives) are known in the pharmaceutical art and can be used in the preparation of the compositions of the present invention. For example, iron oxide can be added to the composition to provide a yellow color.

In one embodiment of the present invention, the inhibitor composition blended with the animal food composition and comprises at least one cyclooxygenase-2 inhibitor in a desired amount and a binding agent, such as polyvinylpyrrolidone. The inhibitor composition may further comprise one or more carrier materials selected from the group consisting of pharmaceutically acceptable diluents, disintegrants, binding agents, wetting agents, and lubricants. In one embodiment, for example, the inhibitor composition comprises one or more carrier materials selected from the group consisting of lactose, sodium lauryl sulfate, croscarmellose sodium, magnesium stearate, and microcrystalline cellulose. In another embodiment, the inhibitor composition comprises lactose monohydrate and croscarmellose sodium. In still another embodiment, the inhibitor composition further comprises one or more of the carrier materials sodium lauryl sulfate, magnesium stearate, and microcrystalline cellulose.

### Preparation of Cyclooxygenase-2 Inhibitors

The cyclooxygenase-2 inhibitor or inhibitors used in the novel food compositions of the present invention can be prepared in the manner set forth in of WO 00/53149.

### Food Compositions

The food compositions and food materials employed in the present invention are not narrowly critical. They may be food compositions ordinarily intended for human consumption or food compositions ordinarily intended for animal consumption, such as pet foods and livestock feed. These compositions are not intended to be restricted by any specific listing of ingredients. The terms "food", "food composition" and "food material" as used in this invention refer to any natural, processed, manufactured or otherwise modified organic material that can be consumed by humans or animals for nourishment. The terms "feed", "animal feed" and "animal food composition" as used in this invention refer to any natural, processed, manufactured or otherwise modified organic material that can be consumed by animals for nourishment.

By way of example and not limitation, the animal food compositions used in the invention comprise commercially available dog foods where the animal to be treated is a dog. Aside from nutrition balancing additives such as vitamins and minerals, or other additives such a preservatives, emulsifiers, and the like, dog foods generally consist of ingredients that may either be termed substantially proteinaceous or ingredients that may be termed substantially farinaceous. Although the following description should not be considered limiting for the purposes of the present invention, the proteinaceous ingredient may be defined as any material having a protein content of at least 15% by weight whereas the farinaceous ingredient may be defined as having a protein content below about 15% by weight and a major fraction of starchy or carbohydrate containing materials.

Examples of proteinaceous materials typically used in commercial pet foods, including dog foods, are vegetable protein meals such as soybean, cottonseed, and peanut; animal proteins such as casein, albumen, and meat tissue including fresh meat; as well as rendered or dried "meals" such as fish meal, poultry meal, meat meal, bone meal, and the like. Other types of proteinaceous materials include microbial proteins such as yeast and other types of protein such as wheat gluten or corn gluten.

Examples of typical farinaceous materials are grains such as corn, milo, alfalfa, wheat, soy hulls and various other grains which are relatively low in protein.

Numerous other materials can be added to dog foods that do not necessarily fall into either the proteinaceous or farinaceous category such as dried whey and other dairy by-products or carbohydrates. The present invention, as noted, is not intended to be limited by specific combinations of ingredients that can be used to formulate a dog food material.

Such dog foods typically are available in the form of dry dog foods, intermediate moisture dog foods, or high moisture dog foods. A dry dog food is generally characterized by a moisture content below about 15% by weight and comprises a mixture of proteinaceous and farinaceous grains and other materials that are extruded and dried to ambient moisture to provide a product that is highly palatable and convenient for a consumer to feed a pet. An intermediate moisture dog food generally has a higher moisture content of about 15% to about 45% by weight. Although such dog food contains ingredients similar to those found in dry dog food, it often also includes fresh meat as an ingredient. Intermediate moisture dog foods typically require the addition of various materials to provide microbiological and antimycotic stability for the product. High moisture dog foods generally have a moisture content exceeding 45% by weight and for the most part contain meat as the primary ingredient. Typically these dog foods are sterilized and canned.

Although the following is not intended to be limiting in that it is not relevant to the function or effectiveness of the cyclooxygenase-2 inhibitor used in the present invention, it should be recognized that the term "dog food composition" or "dog food material" is generally intended to apply to commercially available, nutritionally balanced dog food compositions. These compositions are typically sold in the form of discrete particles of the dog food composition. Dog food compositions meeting this definition may, therefore, be characterized by a minimum protein level required when the dog food composition provides the sole food intake for the dog. Commercially available dry dog food compositions typically have a minimum protein content that is dependent upon the age of the animal to which it is to be fed, or if the animal is mature, whether or not involved in breeding. Thus, while females involved in breeding, or puppies would require a minimum protein content of at least about 20% by weight and preferably about 20% to 25% by weight on a 90% dry matter basis in the composition, dogs not in either of the above two categories would require a minimum protein level of at least about 15% by weight based on a 90% dry matter basis in the composition. These figures are based on the assumption that the dog food composition provides the sole food intake for the dogs and, therefore, the resultant commercial dog food compositions typically contain a minimum protein level of at least about 15% by weight on a 90% dry matter basis in the composition in order to meet the nutritional requirements of any type of dog.

This minimum level of protein in commercially sold dog food compositions is contrasted with commercially available, nutritionally balanced cat food compositions that normally have protein compositions that are somewhat higher than dog foods. For example, cat food compositions that are commercially available typically contain a minimum protein content of at least about 20% by weight on a 90% dry matter basis and usually are substantially above this because cats, when breeding, and kittens require a minimum protein level of at least about 28% by weight on a 90% dry matter basis. Mature cats, on the other hand, not involved in reproduction, require the minimum protein level of at least about 20% by weight on a dry matter basis depending upon the exact type of proteinaceous source employed. Preferably, the protein content will be at least about 30% by weight on a dry matter basis in the product. These figures are based on the assumption that the cat food composition provides the sole food intake for the cats.

As noted above, however, any food composition useful in orally delivering cyclooxygenase-2 inhibitors may be employed, although commercially available food compositions likely will be the most practical and suitable food compositions. Additional nonlimiting animal food compositions that may be employed in the present invention include, but are not limited to, those compositions disclosed in the following patents:
Eichelburg, U.S. Patent 3,997,675;
Weyn, U.S. Patent 4,039,687;
Bone, U.S. Patent 4,039,689;
Prussin, U.S. Patent 4,053,647;
Cante et al., U.S. 4,211,797;
Lazarus et al., U.S. Patent 4,296,132;
Nahm et al., U.S. Patent 4,310,558;
Spradlin et al., U.S. Patent 4,393,085;
Friedman et al., U.S. Patent 4,495,208;
Tonyes et al., U.S. Patent 4,713,250;
Scaglione et al., U.S. Patent 4,735,808;
Hogan et al., U.S. Patent 4,800,093;
Hamilton et al., U.S. Patent 4,886,679;
Likuski et al., U.S. Patent 4,971,820;
Lasater et al., U.S. Patent 5,200,218;
Lanter, U.S. Patent 5,217,740;
Spanier et al., U.S. Patent 5,532,010;
Marino, U.S. Patent 5,552,176;
Matsuura et al., U.S. Patent 5,756,088; and
Ogilvie et al., U.S. Patent 5,776,913.

These patents, including the specific animal food compositions described therein, are expressly incorporated by reference in this application.

In addition, where desirable, the food compositions can be processed and/or packaged to form a distinct three dimensional shape, herein referred to as a "shaped composition". One nonlimiting example of a shaped composition is a food composition processed and packed to form a rectangular stick comprising such food composition using paper wrapping such that the packaged sticks can be placed into box containers (similar to the packaging of sticks of butter). In another example, a moldable food composition is placed inside a packaging material, such as a cylindrical plastic wrapper, with the food composition adopting the shape afforded by that packaging material. Preparing the food composition in the form of a shaped composition allows for greater ease in packing; distributing and administering such food compositions.

Such food compositions also can be processed to provide a dry, brittle form of said food composition, herein referred to as a "brittle food composition", such that the food composition is easily breakable into discrete uniform portions without the use of any tools. This "easily breakable" aspect of a brittle food composition is enhanced by providing linear zones of reduced mechanical strength throughout the brittle food composition. This enables the user to break off discrete quantities of the brittle food composition.

In a particular embodiment, the present invention also provides a food composition comprising one or more visually meterable dose units, each dose unit comprising a cyclooxygenase-2 inhibitor in a therapeutically or prophylactically effective amount for a non-human animal of body weight greater than about 1 kg, the cyclooxygenase-2 inhibitor being substantially homogeneously dispersed in a food material.

A "metered amount" or "metered dosage amount" as used herein refers to a discrete amount of a food composition determined by volumetric measurement of the food composition, wherein said discrete amount is physically separable from said food composition and said food composition contains a substantially uniform amount of a selective cyclooxygenase-2 inhibitor per unit volume of food composition. Said volumetric measurement can be accomplished in any appropriate manner, including, but not limited to, measuring a discrete volume of a granular or particulate food composition using a standard measuring cup, measuring a discrete volume of a food composition having a uniform cross-section that is encased in a cuttable wrapper based upon increments of dosage amount printed on the wrapper itself, or by any other appropriate means.

The term "visually meterable dose unit" as used herein is a metered amount of a food composition wherein said metered amount is determined by observing, usually by sight, the markings on either the package of the food composition or within the food composition itself, and subsequently, if necessary, physically separating the food composition as indicated by the marking or indicia. One nonlimiting example of a visually meterable dose unit is a food composition wherein the metered amount is individually packaged thereby eliminating the need to physically separate the metered amount from the bulk of the food composition.

A cyclooxygenase-2 inhibitor is "substantially homogeneously distributed" or "substantially homogeneously dispersed" in the food composition when the food composition is fully mixed such that the cyclooxygenase-2 inhibitor neither separates into discrete layers in the food composition nor forms concentration gradients within the food composition.

In another embodiment, a cyclooxygenase-2 inhibitor is substantially homogeneously distributed in a food composition and a metered amount of the food composition is administered to a non-human animal. The metered amount provides the animal with a therapeutically or prophylactically effective amount of the cyclooxygenase-2 inhibitor and satisfies the daily recommended caloric intake of animal.

### Additives

The food compositions may further comprise other food additives that are compatible with the cyclooxygenase-2 inhibitor employed. Preferred additives include one or more members of the group selected from antibiotics, antiparasitaries, chemotherapeutics, vitamins, minerals, bactericides, nutrients, additional compatible therapeutic agents (such as diuretics), anti-oxidants (such as butylated hydroxy toluene and butylated hydroxy anisol), chelators (such as EDTA and EGTA), immune system stimulants, appetite stimulants, color enhancers, palatability enhancers and dietary supplements. The additives may be present in conventional dosages known to those of ordinary skill in the art.

### Indicator Substances

The food compositions also may further comprise one or more physiologically acceptable indicator substances that changes the body excretions, such as feces and urine, of the animal in a characteristic manner. For example; 5% iron(II) sulfate can be added to the composition as an indicator. This substance causes a black coloration of the animal feces and makes it possible to determine whether the animal has consumed the composition comprising the cyclooxygenase-2 inhibitor, particularly where the composition is being fed to more than one animal.

The selection of food compositions and cyclooxygenase-2 inhibitors is as previously discussed above. Likewise, the dosage regimen to prevent, give relief from, or ameliorate the condition or disorder preferably corresponds to the daily dosages discussed above, but may be modified in accordance with a variety of factors. These include the type, age, weight, sex, diet, and medical condition of the animal and the severity of the disease. Thus, the dosage regimen actually employed may vary and therefore deviate from the preferred dosage regimen set forth above.

Initial treatment of a animal suffering from a condition or disorder where treatment with a cyclooxygenase-2 inhibitor is indicated can begin with the dosages indicated above. Treatment is generally continued as necessary over a period of several weeks to several months or years until the condition or disorder has been controlled or eliminated. Animals undergoing treatment with the compositions disclosed herein can be routinely monitored by any of the methods well known in the art to determine the effectiveness of therapy. Continuous analysis of such data permits modification of the treatment regimen during therapy so that optimal effective amounts of compositions are administered at any point in time, and so that the duration of treatment can be determined as well. In this way, the treatment regimen/dosing schedule can be rationally modified over the course of therapy so that the lowest amount of cyclooxygenase-2 inhibitor exhibiting satisfactory effectiveness is administered, and so that administration is continued only so long as is necessary to successfully treat the condition or disorder.

### Method For Preparation Of Compositions

Methods for the preparation of animal food compositions comprising at least one cyclooxygenase-2 inhibitor wherein the daily ration of the food composition fed to the animal is sufficient to provide an amount of cyclooxygenase-2 inhibitor to the animal between about 0.1 mg/kg animal body weight to about 15 mg/kg animal body weight are, for example, such methods as:
(1) applying a solution or dispersion of a cyclooxygenase-2 inhibitor in a liquid carrier material to a human food composition or animal food composition capable of absorbing or otherwise retaining the solution or dispersion;
(2) blending unformulated cyclooxygenase-2 inhibitor with a human food composition or animal food composition;
(3) blending the cyclooxygenase-2 inhibitor in combination with one or more carrier materials, such as a binder, with a human food composition or animal food composition; and
(4) dispersing or dissolving the cyclooxygenase-2 inhibitor in a material such as a vegetable oil or an edible fat which is then sprayed or otherwise coated on the animal food composition whereby the cyclooxygenase-2 inhibitor is adsorbed on, coated on or otherwise affixed to the outer surface of the food composition, or is absorbed by the food composition.

In one embodiment, a dog food composition is blended during processing with an inhibitor composition comprising about 1 to about 95 weight percent of cyclooxygenase-2 inhibitor; about 5 to about 99 weight percent of a pharmaceutically acceptably diluent; about 0.5 to about 30 weight percent of a pharmaceutically acceptably disintegrant; and about 0.5 to about 25 weight percent of a pharmaceutically acceptably binding agent. This composition may optionally comprise about 0.25 to about 15 weight percent of a pharmaceutically acceptably wetting agent; and/or about 0.1 to about 10 weight percent of a pharmaceutically acceptably lubricant. The term "weight percent" as used herein means the weight percent of a specified ingredient based upon the total weight of all ingredients of the inhibitor composition. Preferably, the inhibitor composition is added to the food composition at a stage of the process wherein the remaining process steps will not materially degrade the cyclooxygenase-2 inhibitor.

In another embodiment, a dog food composition is blended during processing with an inhibitor composition comprising about 1 to about 95 weight percent of cyclooxygenase-2 inhibitor; about 5 to about 99 weight percent of lactose; about 2 to about 6 weight percent of croscarmellose sodium; and about 0.5 to about 10 weight percent of polyvinylpyrrolidone. This composition may optionally comprise about 0.25 to about 7 weight percent of sodium lauryl sulfate; about 0.1 to about 10 weight percent of magnesium stearate; and/or about 1 to about 99 weight percent of microcrystalline cellulose.

In another embodiment, a dog food composition is blended during processing with an inhibitor composition comprising about 25 to about 85 weight percent of cyclooxygenase-2 inhibitor; about 5 to about 70 weight percent of lactose; about 0.5 to about 7 weight percent of polyvinylpyrrolidone; and about 0.2 to about 5 weight percent of croscarmellose sodium. This composition may optionally comprise about 0.4 to about 6 weight percent of sodium lauryl sulfate; about 0.2 to about 8 weight percent of magnesium stearate; and/or about 0.1 to about 15 weight percent of microcrystalline cellulose.

In another embodiment, an edible fat comprising the cyclooxygenase-2 inhibitor is used to provide an effective and uniform coating of the inhibitor on a dog food composition. The specific type of fat that is suitable for use in this embodiment is not narrowly critical as long as the cyclooxygenase-2 inhibitor is sufficiently soluble in the fat. Typical fats that may be employed include animal fats such as lard and tallow. The particular level of fat employed is dependent upon the nutritional characteristics desired for the dog food and is not narrowly critical to the effectiveness of the cyclooxygenase-2 inhibitor. Typical levels of fat that are employed as a coating are between about 5% and about 20% by weight of the dog food composition.

Preferably, a fat-soluble cyclooxygenase-2 inhibitor is thoroughly mixed with the fat in order to provide a uniform distribution of the inhibitor on the surfaces of the dog food particles. The soluble nature of the cyclooxygenase-2 inhibitor in the fat provides an additional advantage because the uniform application of the inhibitor to the surfaces of the particles is generally assured.

Alternatively, the cyclooxygenase-2 inhibitor could be applied as a separate coating by spraying a dispersion of the inhibitor in another material, for example, a solution of the inhibitor in a vegetable oil, on the particles and then applying a coating of an edible fat over the sprayed particles. It is preferred, however, to simply mix the fat and the cyclooxygenase-2 inhibitor and then apply this mixture to the surfaces of the particles of the dog food compositions. This eliminates the need for two separate spraying steps and provides a uniform and efficient means for applying both materials to the surface of the particles. In any event, the particular manner in which the material is applied is not intended to be critical to the practice of the present invention and effectiveness of the composition can be achieved regardless of the order of addition of fat or cyclooxygenase-2 inhibitor. Typically, the fat, or fat and cyclooxygenase-2 inhibitor mixture, is heated to insure that the fat is completely liquid prior to application by spraying because this facilitates spraying of the fat on the dog food composition. If desired, the sprayed particles can be transferred to a tumbling drum or similar apparatus wherein the coated particles are tumbled repeatedly to improve the uniformity of the coating: The coated dog food particles can then be removed from the tumbling drum and cooled to ambient temperature.

In another embodiment, an edible fat comprising the cyclooxygenase-2 inhibitor is used to provide an effective coating of the inhibitor on a conventional dog biscuit. Because each individual dog biscuit contains a known and uniform amount of the cyclooxygenase-2 inhibitor, the dog owner can administer the desired dosage of inhibitor by feeding the dog an appropriate number of dog biscuits.

In another embodiment, the cyclooxygenase-2 inhibitor is mixed with the ingredients of a conventional dog biscuit during the preparation of the biscuit resulting again in an individual dog biscuit containing a known and uniform amount of the cyclooxygenase-2 inhibitor wherein the dog owner can administer the desired dosage of inhibitor by feeding the dog an appropriate number of dog biscuits.

In another embodiment, the cyclooxygenase-2 inhibitor is mixed with a liquid carrier material in which the inhibitor will dissolve or disperse. The mixture is then applied to a human or animal food composition capable of absorbing the mixture. The liquid carrier material can be, for example, a commercially available vegetable oil such as olive oil. It can be applied to the food composition by any appropriate means including, but not limited to, pouring, brushing or using an eyedropper. Use of an eyedropper, for example, allows for the precise administration of the cyclooxygenase-2 inhibitor since the amount of inhibitor and volume of liquid carrier material are known and can be used to calculate the actual dosage to be administered. The food composition can be any food that will absorb or otherwise retain the mixture such as, but not limited to, breads, cookies and other baked goods. The food composition comprising the cyclooxygenase-2 inhibitor and liquid carrier material is then fed to the animal, particularly a dog or horse.

In another embodiment, the method comprises dissolving or uniformly dispersing a cyclooxygenase-2 inhibitor in a liquid edible material at a temperature below the decomposition point of the cyclooxygenase-2 inhibitor to form a solution or dispersion, and mixing the solution or dispersion with a food material to form a food composition wherein the cyclooxygenase-2 inhibitor is substantially homogeneously distributed.

### Articles of Manufacture

The food compositions disclosed herein are prepared in the form of discrete articles of manufacture. Nonlimiting examples of such articles of manufacture are described below.

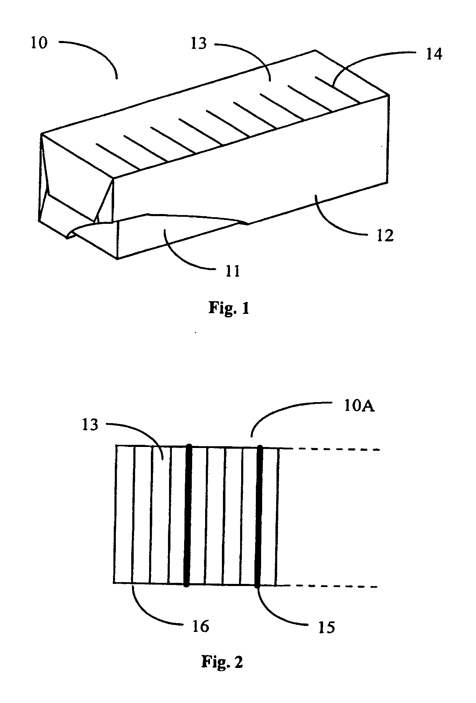

In one embodiment, an article of manufacture **10** of the present invention is illustratively shown in Fig. 1. A shaped, illustratively rectilinear, composition **11** is enclosed in a wrapper **12** composed of a cuttable, printable material. The composition **11** comprises a food material, illustratively a spreadable butter-like material, wherein is substantially homogeneously distributed a cyclooxygenase-2 inhibitor. On at least one face **13** of the wrapper are printed marks **14** that indicate lines along which the article **10** or the shaped composition **11** can be cut to provide a slice containing a metered dose of the cyclooxygenase-2 inhibitor.

In another embodiment, an article of manufacture **10A** of the present invention, similar in many respects to article **10,** is illustratively shown in top view in Fig. 2. In this article the wrapper **12** is marked with major indicia **15** and minor indicia **16,** indicating lines along which the article **10A** can be cut to provide a slice containing respectively a metered large or small dose of the cyclooxygenase-2 inhibitor.

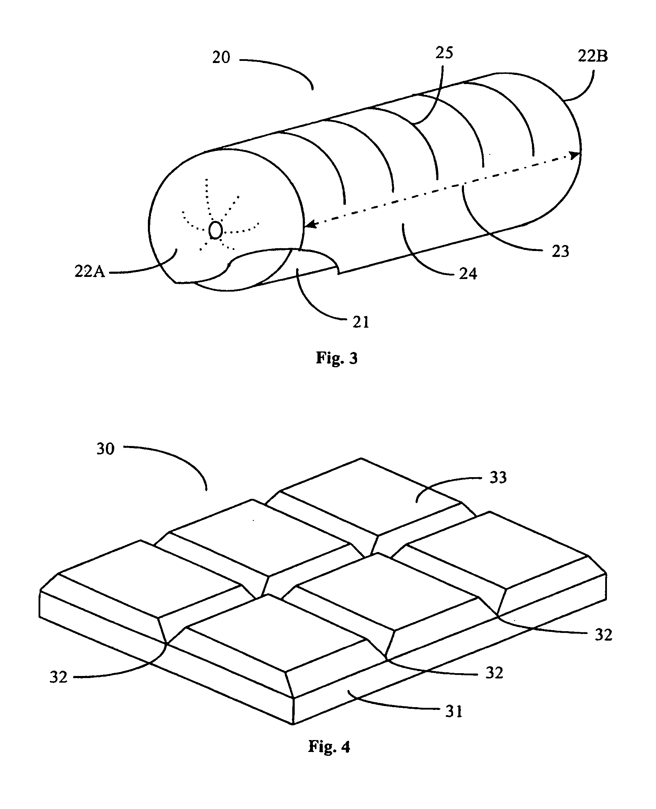

In another embodiment, an article of manufacture **20** of the present invention is illustratively shown in Fig. 3. A shaped, illustratively cylindrical, composition **21,** enclosed in a wrapper **24,** has two substantially planar ends **22A** and **22B** and an elongate dimension **23.** The composition **21** comprises a food material, illustratively a meat-flavored sausage-like material, wherein is substantially homogeneously distributed a cyclooxygenase-2 inhibitor. On the wrapper **24** are printed marks **25** along which the article **20** or the shaped composition **21** can be cut to provide a slice containing a metered dose of the cyclooxygenase-2 inhibitor.

In another embodiment, an article of manufacture **30** of the present invention is illustratively shown in Fig. 4. The article comprises a slab of brittle food material **31,** illustratively a baked material such as a biscuit or cracker, wherein is substantially homogeneously distributed a cyclooxygenase-2 inhibitor. The article is readily breakable, for example by hand, along linear grooves **32** to provide substantially even-sized portions **33** each containing a metered dose of the cyclooxygenase-2 inhibitor. If desired the grooves can be replaced by equivalent means for creating linear zones of reduced mechanical strength, such as linearly aligned perforations or indentations.

Distinct versions of each article of manufacture can be prepared to provide, for example, a low, medium or high dose amount of cyclooxygenase-2 inhibitor per unit volume of food material. Depending upon the animal treated, the severity of the condition treated, and other relevant factors, the animal owner can select a version of the article of manufacture containing the most convenient and appropriate unit dose amount of the cyclooxygenase-2 inhibitor.

### EXAMPLES

The following examples illustrate aspects of the present invention but should not be construed as limitations. The symbols and conventions used in these examples are consistent with those used in the contemporary animal food composition and veterinary literature. Unless otherwise stated, all percentages recited in these examples are weight percents based on total composition weight.

### Example 1: Preparation of Celecoxib Compositions

Three separate Celecoxib compositions having the compositions set forth in Table X-1A are prepared for use in the preparation of the animal food compositions of the succeeding examples:

**TABLE X-1A**

| **INGREDIENT** | **WEIGHT** **FRACTION** **(%)** | | |
|---|---|---|---|
| | **Composition** **1A-1** | **Composition** **1A-2** | **Composition** **1A-3** |
| Celecoxib | 37.04 | 74.07 | 40 |
| Lactose Monohydrate (NF, Ph Eur) | 55.46 | 18.43 | 40.75 |
| Sodium Lauryl Sulfate (NF, Ph Eur) | 3 | 3 | 3 |
| Povidone (K29-32 USP) | 2.5 | 2.5 | 2.5 |
| Croscarmellose Sodium (NF, Ph Eur) | 1 | 1 | 3 |
| Magnesium Stearate (NF, Ph Eur) | 1 | 1 | 0.75 |
| Microcrystalline Cellulose (Avicel PH-102 NF) | 0 | 0 | 10 |
| Total | 100 | 100 | 100 |

Lactose monohydrate is commercially available from Formost Farms, Baraboo, Wisconsin. The Ac-Di-Sol brand of croscarmellose sodium is commercially available from FMC Corporation, Chicago, Illinois. Sodium lauryl sulfate is commercially available from Henkel Corporation, Cincinnati, Ohio. The Povidone brand of polyvinylpyrrolidone is commercially available from International Specialty Products. Magnesium stearate is commercially available from Mallinckrodt Inc., St. Louis, Missouri. The Avicel brand of microcrystalline cellulose is commercially available from FMC Corporation, Philidelphia, Pennsylvania.

An illustrative process for the preparation of composition 1A-1 is described below. Compositions 1A-2 and 1A-3 are prepared in a similar manner.

Milling: The Celecoxib was milled in an impact-type pin mill with counter rotating disks. At mill speeds ranging from about 8960 rpm/5600 rpm to about 11200 rpm/5600 rpm (rotating rpm/counter rotating rpm) particle size varied within relatively narrow ranges (at least 90% of the particles were 30 microns or less in size) suggesting that mill speed is not narrowly critical to the bulk drug micronization process.

Dry Mixing: The Celecoxib, lactose, Povidone and croscarmellose sodium were transferred to a 120 L Niro Fielder PMA-120 high speed granulator and mixed for about 3 minutes at fast chopper and impeller speeds. This dry mixing time provided adequate mixing of Celecoxib with the carrier materials prior to the start of the wet granulation step.

Wet Granulation: Sodium lauryl sulfate (8.1 kg) was dissolved in purified USP water (23.7 kg). This solution was progressively added to the granulator at a rate of about 14 kg/minute. Total granulation time was about 6.5 minutes. During this granulation, the main blade and chopper blade of the granulator were placed on the fast speed setting. The wet granulated mixture was about 8.1 % water by weight.

Drying: The wet granulation was delumped using a Quadro Comil Model 198 S screening mill equipped with rotating impeller and a coarse screen. Wet milling was used to eliminate large material lumps that formed as a by-product of the wet granulation operation. If not removed, these lumps would have prolonged the subsequent fluidized bed drying operation and increased the variation with respect to moisture control. The delumped granulation was transferred to an Aeromatic Fluid Bed Dryer T-8. The inlet air temperature and flow rate were adjusted to about 60°C and about 5000 to 6000 ft³/minute. The granulation was dried in the fluidized bed dryer to reduce the moisture content to between 0.5% to 2.5%. Moisture content was monitored using a Computrac Moisture Analyzer. Drying continued until the loss on drying of the granulation was not more than 1.0%. It may be desirable to combine two or more granulation sections for this drying step and subsequent processing steps.

Dry Milling: The dry granules were passed through a Fluid Air Mill Model 007 impact mill (conventional hammer) equipped with a 0.028 inch to 0.063 inch screen, knives forward, and 2400 rpm speed. Dry milling was used in combination with the wet granulation step to control the final size distribution of the granules.

Blending and Lubrication: The milled granules were then placed in a PK Cross-Flow Blender 75 Cubic Foot diffusion mixer/V- blender. The magnesium stearate was added and the mixture blended for about 5 minutes. The blending time provided blended material that was uniform with respect to the concentration of Celecoxib. Blender rotational speed was 10.6 revolutions per minute. The final blend was used to combine materials from multiple granulation sections into a single uniform mixture and to evenly distribute lubricant into the material.

### Example 2

Each of the dog food compositions 2A-1, 2A-2, 2A-3, 2B-1 and 2B-2 is prepared starting with the ingredients in the proportions set forth in Tables X-2A and X-2B. To this starting mixture is added the Celecoxib composition 1A-1 prepared in Example 1 above which is then thoroughly mixed with the starting ingredients. The amount of Celecoxib composition added to the starting materials is such that a predetermined daily ration of the final dog food composition is sufficient to provide about 4 mg Celecoxib/kg animal body weight to a dog consuming that composition.

This mixture is transferred to a steam conditioner and subjected to steam and moisture in order to adjust the moisture content to between about 20% and 40% by weight. The conditioned mixture is then extruded under conditions of elevated temperature and pressure to form a continuous strand of expanded product that is segmented into discrete particles or pieces by a rotating cutting knife upon exit of the strand from the extruder: The particles are then conveyed to a forced air drying system and the moisture level reduced to below about 10% of the weight. The dried, extruded dog food particles after exit from the forced air oven and prior to cooling are transported from the dryer to a spray chamber by a bulk conveyor. The particles are dropped from the conveyor belt in a sheet and fall through the spray chamber. Spray heads located on both sides of the falling sheet spray a solution of the indicated amount of animal fat on the hot particles as they fall through the spray chamber.

These compositions are then heated to a temperature of about 104°F to facilitate spraying on the hot particles of the dog food composition in the spray chamber. The spray coated dog food particles are collected at the bottom of the spray chamber and transported to a tumbling drum. The tumbling drum is maintained at a temperature above the melting point of the fat and the particles are tumbled until they have a substantially uniform coating of the fat on the surfaces thereof. The coated food particles are then removed from the drum and cooled to ambient temperature. The resultant dried dog food composition has a moisture content of less than about 12% by weight, and a protein content above about 15% by weight on a 90% dry matter basis.

Each of the dog food compositions 2A-1, 2A-2, 2A-3, 2B-1 and 2B-2 also can be prepared as described above, but instead using either unformulated Celecoxib or Celecoxib composition 1A-2 or 1A-3 (prepared in Example 1) in place of Celecoxib composition 1A-1. As above, the amount of Celecoxib added to the starting materials is such that a predetermined daily ration of the final dog food composition is sufficient to provide about 4 mg Celecoxib/kg animal body weight to a dog consuming that composition.

Alternatively, instead of directly mixing the Celecoxib with the starting materials, the Celecoxib can be dispersed or dissolved in the animal fat coating material and then sprayed on the extruded dog food particles in the same manner as the spraying process described above.

**TABLE X-2A**

| **INGREDIENT** | **COMPOSITION** **(% BY WEIGHT)** | | |
|---|---|---|---|
| | **2A-1** | **2A- 2** | **2A-3** |
| Corn | 53.76 | 53.75 | 53.83 |
| Corn Gluten Meal (60% protein) | 12.43 | 12.43 | 12.43 |
| Soybean Meal (49% protein) | 7.83 | 7.83 | 7.83 |
| Salt | 1.00 | 0.80 | |
| Sodium Bicarbonate | 0 | 0.30 | 1.45 |
| Trace Minerals | .20 | .20 | .20 |
| Potassium Bicarbonate | | 1.00 | 1.00 |
| Calcium Carbonate | 1.29 | 1.29 | 1.29 |
| Dicalcium Phosphate | 2.58 | 2.58 | 2.58 |
| Vitamin premix | 0.54 | 0.54 | 0.54 |
| Sucrose | 1.68 | 0.76 | 0.39 |
| Soy Protein Isolate | 10.01 | 10.01 | 9.85 |
| Rice Hulls | 3.50 | 3.50 | 3.60 |
| Concentrated Hydrochloric Acid | 0.17 | 0 | 0 |
| Fat | 5.00 | 5.00 | 5.00 |
| Vitamin A & E oil | .01 | .01 | .01 |

**TABLE X-2B**

| **INGREDIENT** | **COMPOSITION (% BY** **WEIGHT)** | |
|---|---|---|
| | **2B-1** | **2B-2** |
| Corn | 39.49 | 0 |
| Wheat | 0 | 64.27 |
| Corn Gluten Meal (60% protein) | 8.64 | 8.70 |
| Soybean Meal (49% protein) | 18.8 | 6.50 |
| Calcium Chloride | 0 | 0.25 |
| Fish Meal | 0 | 5.00 |
| Meat & Bone Meal | 8.80 | 5.50 |
| Salt | 0.74 | 0.50 |
| Mineral Mixture | 0.20 | 0.20 |
| Whey | 1.47 | 1.07 |
| Choline Chlorides | 0 | 0.27 |
| Lysine | 0.16 | 0.18 |
| Vitamin Premix | 0.55 | 0.55 |
| Fat | 5.49 | 5.00 |
| Vitamin A&E Oil | .01 | 0.01 |
| Defluorinated Phosphate | 0.55 | 0 |
| Rice Hulls | 4.5 | 0 |
| Corn Gluten Fee | 10.6 | 0 |

### Example 3

A filamentous fungal biomass is prepared as set forth below. Specifically, 200 L of soybean whey, which is refrigerated and stored at 7°-10°C to prevent microorganism growth and has a solids level of about 1.5% by weight is pumped into a 300 L stainless steel fermentor ("Chemapee" unit, manufactured by Chemapec, 230 Crossways Park, Woodbury, NY 91797). This unit has means for controlling pH, dissolved oxygen level, agitation and has temperature control. The temperature of the soybean whey is raised to about 121°C over a period of 10 to 20 minutes and held for 15 minutes at 121°C with agitation to sterilize the soybean whey.

In a separate operation, sufficient inoculum for the soybean whey is prepared as follows. To each of seven 2 L Erlenmeyer flasks 400 mL of the soybean whey is added. The whey is sterilized by heating at 121°C for 15 minutes. To one of the seven flasks 1 mL of a suspension of Aspergillus oryzae spores (NRRL 2217) is added. The flask to which the mold is added is then shaken at 400 r.p.m. and maintained at 30°C for a period of 12 to 24 hours or until adequate growth of the mold is achieved. The mold growth is adequate when the culture is whitish with the appearance of applesauce. If inadequate growth has occurred, the media is thin and watery. The culture from the single flask is then divided equally among the six other flasks containing sterilized soybean whey. The flasks are then shaken at 400 r.p.m. and maintained at 30°C for a period of 12 to 24 hours or until adequate growth of the mold is achieved as described above.

The culture obtained from the six flasks is then divided between two 10 L fermentors ("LSL/Biolafitte" units manufactured by LSLBiolafitte, 719 Alexander Rd., Princeton, NH 08540). Each of these fermentors contains 13 L of the sterilized soybean whey, and growth of the mold is then carried out in the fermentor for a period of 20 to 22 hours. During this time the pH of the inoculated medium is maintained at 4.2 ± 0.2, and the dissolved oxygen level at 80% of saturation. The medium is agitated at 350 r.p.m. and maintained at a temperature of 30°C ± 2°C for the noted period of time.

Following elapse of the noted period of time, the contents of the two 20 L fermentors are added to the remaining sterilized soybean whey contained in the 300 L fermentor. Growth of the mold is then allowed to proceed for the period of time under the conditions described above to produce a larger quantity of a filamentous fungal biomass.

The contents of the fermentor are then gravity filtered through cheese cloth to yield a filamentous fungal biomass product having a solids level of about 10% by weight. This wet biomass can be frozen and later used to produce the cat food products described below.

Each of the cat food compositions 3A-1 and 3A-2 set forth in Table X-3A below are prepared by grinding the fresh meat and, in the case of composition 3A-2, the biomass at a temperature of about 30°F through a grinder equipped with a 1/8 inch grinding plate. The ground mixture of fresh meat (and biomass) is placed in a heating unit and heated to a temperature of 120°F. The remainder of the ingredients are then added in the amounts indicated in Table X-3A together with the Celecoxib composition 1A-1 prepared in Example 1 above. The resulting mixture is thoroughly blended. The amount of Celecoxib composition added to the starting materials is such that a predetermined daily ration of the final cat food composition is sufficient to provide about 2 mg Celecoxib/kg animal body weight to a cat consuming that composition.

The mixture is maintained at a temperature of 120°F for 45 minutes and conveyed to can filling equipment at which point the cans containing the mixture are filled and sealed. The cans are placed in baskets which are lowered into vertical retorts operated at a temperature of about 245°-250°F. The cans are held at this temperature for about 65 minutes. The cans are cooled, rinsed and allowed to dry.

**TABLE X-3A**

| **INGREDIENT** | **COMPOSITION (% BY** **WEIGHT)** | |
|---|---|---|
| | **3A-1** | **3A-2** |
| Liver Digest | 2.0 | 2.0 |
| Whole Chicken Carcass | 55.0 | 50.0 |
| Filamentous Fungal Biomass From Above | 0 | 5.0 |
| Beef Lungs | 10.0 | 10.0 |
| Liver | 3.0 | 3.0 |
| Poultry Meal | 5.0 | 5.0 |
| Vegetable Gums | 1.0 | 1.0 |
| Vitamin and Minerals | 1.2 | 1.2 |
| Water | 22.8 | 22.8 |
| | 100.0% | 100.0% |

A filamentous fungal biomass is produced as described above except that 0.5% by weight/volume of the whey of ground corn is added to the whey before inoculation with the *A. oryzae.* The biomass harvested from the process has a solids level of about 5% by weight. The cat food compositions 3B-1, 3B-2 and 3B-3 set forth in Table X-3B below are prepared by grinding the fresh meat and, in the case of compositions 3B-2 and 3B-3, the biomass at a temperature of about 30°F through a grinder equipped with a 1/8 inch grinding plate. The ground mixture of fresh meat and biomass is placed in a heating unit and heated to a temperature of 120°F. The remainder of the ingredients are then added in the amounts indicated in Table X-3B together with the Celecoxib composition 1A-1 prepared in Example 1 above. The resulting mixture is thoroughly blended. The amount of Celecoxib composition added to the starting materials is such that a predetermined daily ration of the final cat food composition is sufficient to provide about 2 mg Celecoxib/kg animal body weight to a cat consuming that composition.

The temperature of 120°F is maintained for 1 hour for compositions 3B-1 and 3B-2, and 4 hours for composition 3B-3. The mixture for each product is conveyed to can filling equipment at which point the cans containing the mixture are filled and sealed. The cans are then placed in baskets and lowered into vertical retorts operated at a temperature of about 245°-250°F. The cans are held at this temperature for about 65 minutes. The cans are cooled, rinsed, and allowed to dry.

**TABLE X-3B**

| **INGREDIENT** | **COMPOSITION** **(% BY WEIGHT)** | | |
|---|---|---|---|
| | **3B-1** | **3B-2** | **3B-3** |
| Liver digest | 2.0 | 2.0 | 2.0 |
| Whole Chicken Carcass | 55.0 | 12.0 | 12.0 |
| Beef Lungs | 10.0 | 0 | 0 |
| Fungal Biomass | 0 | 56.8 | 56.8 |
| Liver | 3.0 | 4.0 | 4.0 |
| Poultry Meal | 5.0 | 6.0 | 6.0 |
| Soy Meal | 0 | 6.0 | 6.0 |
| Water | 22.3 | 8.0 | 8.0 |
| Vitamins and Minerals | 1.2 | 1.2 | 1.2 |
| Gum Premix | 1.0 | 0 | 0 |
| Ground Corn | | 2.0 | 2.0 |
| Wheat Midds | 0 | 2.0 | 2.0 |
| | 100% | 100% | 100% |

Each of the cat food compositions 3A-1, 3A-2, 3B-1, 3B-2 and 3B-3 also can be prepared as described above, but instead using either unformulated Celecoxib or Celecoxib composition 1A-2 or 1A-3 (prepared in Example 1) in place of Celecoxib composition 1A-1. As above, the amount of Celecoxib added to the starting materials is such that a predetermined daily ration of the final cat food composition is sufficient to provide about 2 mg Celecoxib/kg animal body weight to a cat consuming that composition.

### Example 4

A dog food composition is prepared starting with the ingredients in the proportions set forth in Table X-4A. To this starting mixture is added the Celecoxib composition 1A-1 prepared in Example 1 above which is then thoroughly mixed with the starting ingredients. The amount of Celecoxib composition added to the starting materials is such that a predetermined daily ration of the final dog food composition is sufficient to provide about 3 mg Celecoxib/kg animal body weight to a dog consuming that composition.

**TABLE X-4A**

| **INGREDIENT** | **COMPOSITION (% BY WEIGHT)** |
|---|---|
| Ground yellow corn | 41 |
| Ground whole wheat | 4.3 |
| Corn gluten feed | 4 |
| Corn gluten meal (60% protein) | 9.5 |
| Wheat germ | 0.5 |
| Soybean meal | 14 |
| Meat and bone meal | 18.4 |
| Salt | 0.3 |
| Minerals and vitamins | 1.5 |

The mixture is transferred to a steam conditioner and subjected to steam and moisture in order to adjust the moisture content to between about 20% and about 40% by weight. The conditioned mixture is then extruded under conditions of elevated temperature and pressure to form a continuous strand of expanded product that is segmented into discrete particles or pieces by a rotating cutting knife upon exit of the strand from the extruder. The particles are then conveyed to a forced air drying system and the moisture level reduced to below about 10% by weight. If an intermediate moisture composition is to be produced, this forced air drying step is omitted. The dried, extruded dog food particles after exit from the forced air oven and prior to cooling are transported from the drier to a spray chamber by a bulk conveyor. The particles are dropped from the conveyor belt in a sheet and fall through the spray chamber. Spray heads located on both sides of the falling sheet spray a solution of about 6.5% animal fat on the hot particles as they fall through the spray chamber.

The mixture is heated to a temperature of about 140°F to facilitate spraying on the hot particles of the dog food composition in the spray chamber. The spray coated dog food particles are collected at the bottom of the spray chamber and transported to a tumbling drum. The tumbling drum is maintained at a temperature above the melting point of the fat and the particles are tumbled until they have a substantially uniform coating of the fat on the surfaces thereof. The coated food particles are removed from the drum and cooled to ambient temperature. The resulting dog food composition has a moisture content of less than about 12% by weight (or about 15% to about 45% by weight for the intermediate moisture composition), and a protein content of about 15% by weight on a 90% dry matter basis.

The dog food composition of this Example also can be prepared as described above, but instead using either unformulated Celecoxib or Celecoxib composition 1A-2 or 1A-3 (prepared in Example 1) in place of Celecoxib composition 1A-1. As above, the amount of Celecoxib added to the starting materials is such that a predetermined daily ration of the final dog food composition is sufficient to provide about 3 mg Celecoxib/kg animal body weight to a dog consuming that composition.

Alternatively, instead of directly mixing the Celecoxib with the starting materials, the Celecoxib can be dispersed or dissolved in the animal fat coating material and then sprayed on the extruded dog food particles in the same manner as the spraying process described above.

### Example 5

Dog food composition 5A-1 is prepared using soybean meal, weighing 190 pounds after oil extraction by hexane, as one of the starting materials. The soybean meal preferably has a protein content of about 49% by weight of the meal, and a fat content of about 0.5% by weight. The soybean meal is mixed with 10 pounds of meat and bone meal, about 182 grams of sulfur, an extrusion aid, is added to the mixture as well as 9 grams of a yellow color, 18 grams of a red color, and 32 grams of a brown color. The mixture is then fed into a preconditioner where about 20 pounds of water and steam is admixed, and then into a conventional extrusion device having steam and water jackets. The screw in the extruder is rotated at 150 rpm. The mixture is mechanically worked within the extruder at a temperature of around 300°F, with the pressure varying somewhat but being generally about 300 psig. The material is continuously passed through the extruder, passing through the elongated tube and out an extruder nozzle having a size of 3/8 x 1/8 inch. The reaction time of the material within the extruder is about 30 seconds. The mixture is ejected from the nozzle in a continuous stream, and cut. The coherent fibrous structure of the material is expanded upon passage through the nozzle to form a porous structure. The product, when removed, has a fibrous meatlike texture. The product is dried to a moisture content of about 8%.

Dog food composition 5B-1 is prepared in a manner similar to that described for dog food composition. 5A-1 above, but green color is provided in the fibrous food pieces by the addition of pea flour and a green coloring dye. About 179 pounds of solvent extractant soybean meal having 49% protein is mixed with 20 pounds of pea flour, about 182 grams of sulfur, and 38 grams of a green coloring dye. The mixture is then placed in a preconditioner with about 20 pounds of water. It is extruded in a conventional extruder cooker at conditions recited above for dog food composition 5A-1. The resulting green colored fibrous food pieces are dried to a moisture content of about 9%.

Dog food composition 5C-1 is prepared starting with one hundred pounds of a nutritionally balanced farinaceous-proteinaceous material employed as the basal matrix and having the composition set forth in Table X-5C below.

**TABLE X-5C**

| **INGREDIENT** | **COMPOSITION (% BY WEIGHT)** |
|---|---|
| Ground Corn | 31 |
| Wheat | 20 |
| Whole Oats | 5 |
| Corn Gluten Feed | 8 |
| Corn Gluten Meal | 10 |
| Soybean Meal | 5 |
| Meat & Bone Meal | 18 |
| Vitamin & Mineral Supplements | 3 |

Dog food composition 5C-1 is prepared by thoroughly mixing the above basal matrix formulation with (a) dog food compositions 5A-1 and 5B-1 and #4 brewers rice in a proportion so that there is about 17% by weight composition 5A-1, about 3% by weight composition 5B-1, and about 5% by weight rice pieces based on the weight of the basal matrix formulation, and (b) the Celecoxib composition 1A-1 prepared in Example 1. The amount of Celecoxib composition added is such that a predetermined daily ration of the final dog food composition is sufficient to provide about 2 mg Celecoxib/kg animal body weight to a dog consuming that composition.

Enough water is added to bring the moisture content of the mixture to 25% by weight. A conventional extrusion device is used with water being supplied to the front and rear jackets to maintain an exit water temperature of 160° to 200°F. The cooling water at room temperature is constantly passed through both jackets. The opening in the restraining plate is 3/8 inch in diameter, with the screw being rotated at 150 rpm. The mixture is mechanically worked within the extruder at a temperature of around 250°F, with the pressure varying, but being generally about 200 psig. The material is continuously passed through the extruder, passing through the elongated tube and out a rectangular extruder nozzle having a size of 3/8 x ½ inch. The retention time of the material within the extruder is about 30 seconds. The mixture is ejected from the nozzle in a continuous stream and cut and coated with about 6% animal fat. The final product is dried to a moisture content of about 10% by weight.

Dog food composition 5D-1 is prepared in a similar manner starting with the basal matrix and having the composition set forth in Table X-5D below.

**TABLE X-5D**

| **INGREDIENT** | **COMPOSITION (% BY WEIGHT)** |
|---|---|
| Corn | 13 |
| Rice | 24 |
| Wheat | 35 |
| Corn Gluten Meal | 14 |
| Meat & Bone Meal | 5 |
| Flavor, Vitamin and Mineral Supplements | 9 |

The meal is ground and passed through an extruder cooker having an elevated temperature of about 275°F and pressure of about 250 psig. Water is added to the extruder jackets for temperature control. The product is extruded through a nozzle having the size of 3/8 to ½ inch into flakes and dried to a moisture content of about 10%. This basal matrix is then ground through a 4/64 Hammermill® screen and mixed with (a) the dog food compositions 5A-1 and 5B-1 and a #4 brewers rice in proportions of 17% composition 5A-1, 3% composition 5B-1 and 5% rice, based on the weight of the basal matrix, and (b) the Celecoxib composition 1A-1 prepared in Example 1. The amount of Celecoxib composition added is such that a predetermined daily ration of the final dog food composition is sufficient to provide about 2 mg Celecoxib/kg animal body weight to a dog consuming that composition.

This mixture is then preconditioned in a California pellet mill by steam and water injection so that the moisture content is brought up to 25% by weight. The conditioned meal is then passed into the rolls of the pellet mill, and is formed through a 5/8 inch x 5/8 inch dye. It is then dried to a moisture content of 6% and sprayed with about 6% animal fat and flavoring agents.

Each of the dog food compositions 5C-1 and 5D-1 also can be prepared as described above, but instead using either unformulated Celecoxib or Celecoxib composition 1A-2 or 1A-3 (prepared in Example 1) in place of Celecoxib composition 1A-1. As above, the amount of Celecoxib added to the starting materials is such that a predetermined daily ration of the final dog food composition is sufficient to provide about 2 mg Celecoxib/kg animal body weight to a dog consuming that composition.

Alternatively, instead of directly mixing the Celecoxib with the starting materials, the Celecoxib can be dispersed or dissolved in the animal fat coating material and then sprayed on the extruded dog food particles in the same manner as the spraying process described above.

### Example 6

Composition 6A-1 disclosed in Table X-6A is a high moisture feed for confined animals, such as dogs and cats being shipped, and is prepared as set forth below. Gum A is a blend of carrageenan and locust bean gums available under the trademark "Colloid Cleartic". Both twenty and four hundred pound batches are made using the weight percentages listed in Table X-6A. To the water, the Colloid Cleartic, vitamin premix and preservatives are added in a Lab Myers mixer, and the mixture is heated to 180°F. The temperature of the mix is maintained, with stirring, for three minutes to form a solution. The solution is then allowed to cool, and when the temperature drops to 160°F the rice flour, protein and flavoring agent are added together with the Celecoxib composition 1A-1 prepared in Example 1. The resulting mixture is thoroughly blended. The amount of Celecoxib composition added is such that a predetermined daily ration of the final animal food composition is sufficient to provide about 2 mg Celecoxib/kg animal body weight to an animal consuming that composition. The protein is an isolated soy protein available as "Supro 620". The mixture remains fluid as long as it is held at a temperature between 140° and 160°F. This extends the gelation reaction time, the period prior to the formation of a solid, beyond 30 minutes, extending the period for packaging.

**TABLE X-6A**

| **INGREDIENT** | **COMPOSITION 6A-1** **(% BY WEIGHT)** |
|---|---|
| Rice Flour | 11.100 |
| Corn Carrier | 1.000 |
| Gum A | 1.000 |
| Flavor | 0.300 |
| Protein | 3.400 |
| Plain Salt | 0.100 |
| Dicalcium Phosphate | 4.300 |
| Citric Acid | 0.500 |
| Potassium Sorbate | 1.000 |
| Propionic Acid | 0.500 |
| Fumaric Acid | 1.500 |
| Vitamins & Minerals | 0.289 |
| Water (to balance) | 75.011 |
| | 100.000 |

Animal food composition 6B-1 is prepared in the same manner as animal food composition 6A-1 except that the starting materials employed are those disclosed in Table X-6B instead of those disclosed in Table X-6A.

**TABLE X-6B**

| **INGREDIENT** | **COMPOSITION 6B-1** **(% BY WEIGHT)** |
|---|---|
| Gum A | 1.0 |
| Vitamin Premix | 0.38 |
| Citric acid | 0.5 |
| Propionic acid | 0.5 |
| Potassium sorbate | 1.0 |
| Fumaric acid | 1.5 |
| Flavor | 0.3 |
| Supro 620 | 3.9 |
| Rice flour | 15.92 |
| Water (to balance) | 75.00 |
| | 100.00 |

Dog food composition 6C-1 disclosed in Table X-6C is prepared in the same manner as dog food composition 6A-1 with the modifications discussed below. The water and gum are heated to 180°F and held for three minutes. All ingredients except rice flour, sucrose or dextrin are then added. The product is cooled to 165°F. Either rice flour, sucrose or dextrin is then added together with the Celecoxib. The product is then mixed, poured into 16 fluid ounce trays and sealed.

**TABLE X-6C**

| **INGREDIENT** | **COMPOSITION 6C** **(PERCENT BY WEIGHT)** |
|---|---|
| Gum A | 1.0 |
| Fumaric acid | 1.5 |
| Potassium sorbate | 1.0 |
| Citric acid | 0.5 |
| Propionic acid | 0.5 |
| Flavor | 0.3 |
| Dicalcium phosphate | 0.8 |
| Supro 620 | 3.6 |
| Rice flour or sucrose or dextrin | 15.3 |
| Vitamin premix | 0.5 |
| Water (to balance) | 75.0 |
| | 100.0 |

### Example 7

A 15-year old, black Labrador Retriever, mixed-heritage, neutered-male dog weighing about 25 kg has a history of arthritic-related symptoms that makes it difficult, or impossible, for the dog to stand, walk, or ascend or descend stairs. This dog is treated with a commercially-available drug therapy (Rimadyl carprofan) for a period of nine months without improvement noticed in the dog's arthritic-related symptoms and with some worsening of such symptoms noted over the nine-month period. Over a subsequent period of six months, this dog is treated with a cyclooxygenase-2 inhibitor in the manner described as follows.

About 125 mg of a 95% pure, selective cyclooxygenase-2 inhibitor in white crystalline powder form is dispersed in a teaspoon of olive oil which is then spread over the surface of a single slice of commercially-available wheat bread. The bread is cut into quarters, all of which is then fed to the dog. The dog is observed to consume the entire slice of bread. Within ten (10) days of initial feeding of this dog, as described above, there is observed noticeable improvement in alleviation of arthritic-related symptoms. For example, the dog is observed to walk easily, descend stairs, gain about 4 kg in weight, and be less lethargic in day-to-day activities.

As various changes could be made in the above compositions and methods without departing from the scope of the invention, it is intended that all matter contained in the above description be interpreted as illustrative and not in a limiting sense. All patent documents listed herein are incorporated by reference.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. An article of manufacture comprising a shaped composition having two substantially planar ends, an elongate dimension substantially orthogonal to the ends and a substantially uniform cross-sectional area, the shaped composition comprising a food material having substantially homogeneously distributed therein a selective cyclooxygenase-2 inhibitor, the shaped composition being packaged in a cuttable wrapping material having printed thereon marks at equal spacing along the elongate dimension, said marks corresponding to increments of dosage amount of the cyclooxygenase-2 inhibitor contained in portions of the shaped composition defined by the marks,
wherein said cyclooxygenase-2 inhibitor is selected from a class of compounds of the following formula: 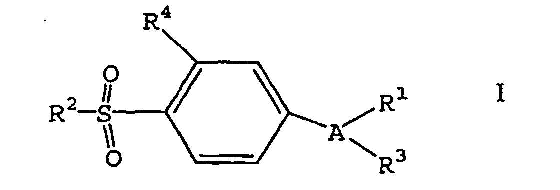 wherein A is a 5- or 6-member ring substituent selected from partially unsaturated or unsaturated heterocyclo and carbocyclic rings;
wherein R¹ is cyclohexyl or phenyl optionally substituted with one, two or three radicals selected from C₁₋₂ alkyl, C₁₋₂ haloalkyl, cyano, carboxyl, C₁₋₂ alkoxycarbonyl, hydroxyl, C₁₋₂ hydroxyalkyl, C₁₋₂ haloalkoxy, amino, C₁₋₂ alkylamino, phenylamino, nitro, C₁₋₂ alkoxy-C₁₋₂-alkyl, C₁₋₂ alkylsulfinyl, halo, C₁₋₂ alkoxy and C₁₋₂ alkylthio;
wherein R² is methyl or amino;
wherein R³ is a radical selected from halo, C₁₋₂ alkyl, alkenyl, alkynyl, oxo, cyano, carboxyl, cyanoalkyl, heterocyclyloxy, C₁₋₂ alkyloxy, alkylthio, alkylcarbonyl, cycloalkyl, phenyl, C₁₋₂ haloalkyl, heterocyclo, cycloalkenyl, phenylalkyl, heterocyclylalkyl, alkylthioalkyl, C₁₋₂ hydroxyalkyl, alkoxycarbonyl, phenylcarbonyl, phenylalkylcarbonyl, phenylalkenyl, alkoxyalkyl, phenylthioalkyl, phenyloxyalkyl, phenylalkylthioalkyl, phenylalkoxyalkyl, alkoxyphenylalkoxyalkyl, alkoxycarbonylalkyl, aminocarbonyl, aminocarbonylalkyl, alkylaminocarbonyl, N-phenylaminocarbonyl, N-alkyl-N-phenylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-phenylaminoalkyl, N-phenylalkylaminoalkyl, N-alkyl-N-phenylalkylaminoalkyl, N-alkyl-N-phenylaminoalkyl, phenyloxy, phenylalkoxy, phenylthio, phenylalkylthio, alkylsulfinyl, alkylsulfonyl, aminosulfonyl, alkylaminosulfonyl, N-phenylaminosulfonyl, phenylsulfonyl and N-alkyl-N-phenylaminosulfonyl; and
wherein R⁴ is hydrido or fluoro;
or a pharmaceutically-acceptable salt thereof, and
wherein each increment of dosage amount corresponds to a portion of said shaped composition containing said selective cyclooxygenase-2 inhibitor in an amount therapeutically or prophylactically effective for a non-human animal of body weight greater than about 1 kg.

2. An article of manufacture comprising a shaped composition that comprises a brittle food material having substantially homogeneously distributed therein a selective cyclooxygenase-2 inhibitor, the shaped composition having means for providing linear zones of reduced mechanical strength permitting breakage into substantially evenly sized portions each containing a metered dosage amount of the cyclooxygenase-2 inhibitor,
wherein said cyclooxygenase-2 inhibitor is selected from a class of compounds of the following formula: 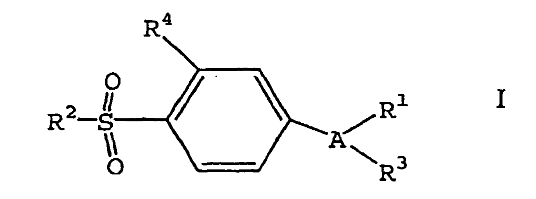 wherein A is a 5- or 6-member ring substituent selected from partially unsaturated or unsaturated heterocyclo and carbocyclic rings;
wherein R¹ is cyclohexyl or phenyl optionally substituted with one, two or three radicals selected from C₁₋₂ alkyl, C₁₋₂ haloalkyl, cyano, carboxyl, C₁₋₂ alkoxycarbonyl, hydroxyl, C₁₋₂ hydroxyalkyl, C₁₋₂ haloalkoxy, amino, C₁₋₂ alkylamino, phenylamino, nitro, C₁₋₂ alkoxy-C₁₋₂-alkyl, C₁₋₂ alkylsulfinyl, halo, C₁₋₂ alkoxy and C₁₋₂ alkylthio;
wherein R² is methyl or amino;
wherein R³ is a radical selected from halo, C₁₋₂ alkyl, alkenyl, alkynyl, oxo, cyano, carboxyl, cyanoalkyl, heterocyclyloxy, C₁₋₂ alkyloxy, alkylthio, alkylcarbonyl, cycloalkyl, phenyl, C₁₋₂ haloalkyl, heterocyclo, cycloalkenyl, phenylalkyl, heterocyclylalkyl, alkylthioalkyl, C₁₋₂ hydroxyalkyl, alkoxycarbonyl, phenylcarbonyl, phenylalkylcarbonyl, phenylalkenyl, alkoxyalkyl, phenylthioalkyl, phenyloxyalkyl, phenylalkylthioalkyl, phenylalkoxyalkyl, alkoxyphenylalkoxyalkyl, alkoxycarbonylalkyl, aminocarbonyl, aminocarbonylalkyl, alkylaminocarbonyl, N-phenylaminocarbonyl, N-alkyl-N-phenylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-phenylaminoalkyl, N-phenylalkylaminoalkyl, N-alkyl-N-phenylalkylaminoalkyl, N-alkyl-N-phenylaminoalkyl, phenyloxy, phenylalkoxy, phenylthio, phenylalkylthio, alkylsulfinyl, alkylsulfonyl, aminosulfonyl, alkylaminosulfonyl, N-phenylaminosulfonyl, phenylsulfonyl and N-alkyl-N-phenylaminosulfonyl; and
wherein R⁴ is hydrido or fluoro;
or a pharmaceutically-acceptable salt thereof
and wherein each portion of said shaped composition contains said selective cyclooxygenase-2 inhibitor in an amount therapeutically or prophylactically effective for a non-human animal of body weight greater than about 1 kg.

3. An article of manufacture of claims 1 to 2 wherein said cyclooxygenase-2 inhibitor is selected from Celecoxib, Deracoxib, Rofecoxib and Valdecoxib.

4. An article of manufacture of claims 1 to 2 wherein said cyclooxygenase-2 inhibitor is Deracoxib.

5. An article of manufacture of claims 1 to 2 wherein each portion of said shaped composition contains said selective cyclooxygenase-2 inhibitor in an amount therapeutically or prophylactically effective for a non-human animal of body weight greater than about 2 kg to about 70 kg.

6. An article of manufacture of claims 1 to 2 wherein each portion of said shaped composition contains said selective cyclooxygenase-2 inhibitor in an amount therapeutically or prophylactically effective for a non-human animal of body weight greater than about 50 kg to about 1500 kg.

7. An article of manufacture of claims 1 to 2 wherein each portion of said shaped composition contains said selective cyclooxygenase-2 inhibitor in an amount therapeutically or prophylactically effective for a dog.

8. An article of manufacture of claims 1 to 2 wherein each portion of said shaped composition contains said selective cyclooxygenase-2 inhibitor in an amount therapeutically or prophylactically effective for a horse.

9. An article of manufacture of claims 1 to 2 wherein each portion of said shaped composition contains a metered dosage amount of said selective cyclooxygenase-2 inhibitor for administration to a non-human animal that is between about 0.1 mg/kg animal body weight to about 15 mg/kg animal body weight.

10. An article of manufacture of claims 1 to 2 wherein each portion of said shaped composition contains a metered dosage amount of said selective cyclooxygenase-2 inhibitor for administration to a non-human animal that is between about 0.5 mg/kg animal body weight to about 10 mg/kg animal body weight.

## Patentansprüche

1. Herstellungsgegenstand umfassend eine geformte Zusammensetzung, die zwei im Wesentlichen ebene Enden, eine im Wesentlichen zu den Enden orthogonale ausgedehnte Dimension und eine im Wesentlichen gleichförmige Querschnittsfläche aufweist, wobei die geformte Zusammensetzung ein Nahrungsmittelmaterial umfasst, das im Wesentlichen homogen darin verteilt einen selektiven Cyclooxygenase-2-Inhibitor aufweist, wobei die geformte Zusammensetzung in einem schneidbaren Hüllmaterial verpackt ist, das in gleich großen Abständen entlang der ausgedehnten Dimension aufgedruckte Markierungen aufweist, wobei die Markierungen Inkrementen der Dosierungsmenge des Cyclooxygenase-2-Inhibitors entsprechen, der in durch die Markierungen definierten Teilen der geformten Zusammensetzung enthalten ist,
wobei der Cyclooxygenase-2-Inhibitor ausgewählt ist aus einer Klasse an Verbindungen der folgenden Formel: 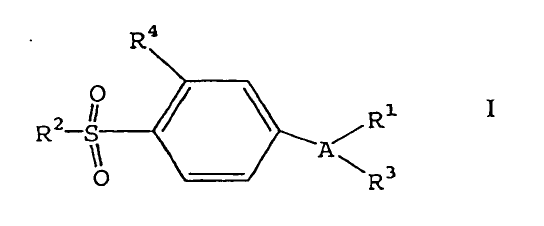 wobei A ein 5- oder 6-gliedriger Ringsubstituent, ausgewählt aus teilweise ungesättigten oder ungesättigten heterocyclischen und carbocyclischen Ringen, ist;
wobei R¹ Cyclohexyl oder Phenyl, gegebenenfalls substituiert mit einem, zwei oder drei Resten, ausgewählt aus C₁₋₂-Alkyl, C₁₋₂-Halogenalkyl, Cyano, Carboxyl, C₁₋₂-Alkoxycarbonyl, Hydroxyl, C₁₋₂-Hydroxyalkyl, C₁₋₂-Halogenalkoxy, Amino, C₁₋₂-Alkylamino, Phenylamino, Nitro, C₁₋₂-Alkoxy-C₁₋₂-Alkyl, C₁₋₂-Alkylsulfinyl, Halogen, C₁₋₂-Alkoxy und C₁₋₂-Alkylthio, ist;
wobei R² Methyl oder Amino ist;
wobei R³ ein Rest, ausgewählt aus Halogen, C₁₋₂-Alkyl, Alkenyl, Alkinyl, Oxo, Cyano, Carboxyl, Cyanoalkyl, Heterocyclyloxy, C₁₋₂-Alkyloxy, Alkylthio, Alkylcarbonyl, Cycloalkyl, Phenyl, C₁₋₂-Halogenalkyl, Heterocyclo, Cycloalkenyl, Phenylalkyl, Heterocyclylalkyl, Alkylthioalkyl, C₁₋₂-Hydroxyalkyl, Alkoxycarbonyl, Phenylcarbonyl, Phenylalkylcarbonyl, Phenylalkenyl, Alkoxyalkyl, Phenylthioalkyl, Phenyloxyalkyl, Phenylalkylthioalkyl, Phenylalkoxyalkyl, Alkoxyphenylalkoxyalkyl, Alkoxycarbonylalkyl, Aminocarbonyl, Aminocarbonylalkyl, Alkylaminocarbonyl, N-Phenylaminocarbonyl, N-Alkyl-N-Phenylaminocarbonyl, Alkylaminocarbonylalkyl, Carboxyalkyl, Alkylamino, N-Arylamino, N-Aralkylamino, N-Alkyl-N-Aralkylamino, N-Alkyl-N-Arylamino, Aminoalkyl, Alkylaminoalkyl, N-Phenylaminoalkyl, N-Phenylalkylaminoalkyl, N-Alkyl-N-Phenylalkylaminoalkyl, N-Alkyl-N-Phenylaminoalkyl, Phenyloxy, Phenylalkoxy, Phenylthio, Phenylalkylthio, Alkylsulfinyl, Alkylsulfonyl, Aminosulfonyl, Alkylaminosulfonyl, N-Phenylaminosulfonyl, Phenylsulfonyl und N-Alkyl-N-Phenylaminosulfonyl, ist; und
wobei R⁴ Hydrido oder Fluor ist;
oder einem pharmazeutisch annehmbaren Salz davon, und
wobei jedes Inkrement der Dosierungsmenge einem Teil der geformten Zusammensetzung entspricht, der den selektiven Cyclooxygenase-2-Inhibitor in einer für ein nicht-menschliches Tier mit Körpergewicht größer als etwa 1 kg therapeutisch oder prophylaktisch wirksamen Menge enthält.

2. Herstellungsgegenstand, umfassend eine geformte Zusammensetzung, die ein brüchiges Nahrungsmittelmaterial umfasst, das im Wesentlichen homogen darin verteilt eine selektiven Cyclooxygenase-2-Inhibitor aufweist, wobei die geformte Zusammensetzung Mittel zum Bereitstellen linearer Zonen reduzierter mechanischer Festigkeit aufweist, die das Brechen in Teile von im Wesentlichen gleichmäßiger Größe, die jeweils eine abgemessene Dosierungsmenge des Cyclooxygenase-2-Inhibitors enthalten, erlauben,
wobei der Cyclooxygenase-2-Inhibitor ausgewählt ist aus einer Klasse an Verbindungen der folgenden Formel: 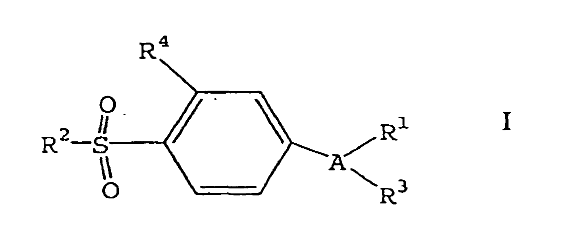 wobei A ein 5- oder 6-gliedriger Ringsubstituent, ausgewählt aus teilweise ungesättigten oder ungesättigten heterocyclischen und carbocyclischen Ringen, ist;
wobei R¹ Cyclohexyl oder Phenyl, gegebenenfalls substituiert mit einem, zwei oder drei Resten, ausgewählt aus C₁₋₂-Alkyl, C₁₋₂-Halogenalkyl, Cyano, Carboxyl, C₁₋₂-Alkoxycarbonyl, Hydroxyl, C₁₋₂-Hydroxyalkyl, C₁₋₂-Halogenalkoxy, Amino, C₁₋₂-Alkylamino, Phenylamino, Nitro, C₁₋₂-Alkoxy-C₁₋₂-Alkyl, C₁₋₂-Alkylsulfinyl, Halogen, C₁₋₂-Alkoxy und C₁₋₂-Alkylthio, ist;
wobei R² Methyl oder Amino ist;
wobei R³ ein Rest, ausgewählt aus Halogen, C₁₋₂-Alkyl, Alkenyl, Alkinyl, Oxo, Cyano, Carboxyl, Cyanoalkyl, Heterocyclyloxy, C₁₋₂-Alkyloxy, Alkylthio, Alkylcarbonyl, Cycloalkyl, Phenyl, C₁₋₂-Halogenalkyl, Heterocyclo, Cycloalkenyl, Phenylalkyl, Heterocyclylalkyl, Alkylthioalkyl, C₁₋₂-Hydroxyalkyl, Alkoxycarbonyl, Phenylcarbonyl, Phenylalkylcarbonyl, Phenylalkenyl, Alkoxyalkyl, Phenylthioalkyl, Phenyloxyalkyl, Phenylalkylthioalkyl, Phenylalkoxyalkyl, Alkoxyphenylalkoxyalkyl, Alkoxycarbonylalkyl, Aminocarbonyl, Aminocarbonylalkyl, Alkylaminocarbonyl, N-Phenylaminocarbonyl, N-Alkyl-N-Phenylaminocarbonyl, Alkylaminocarbonylalkyl, Carboxyalkyl, Alkylamino, N-Arylamino, N-Aralkylamino, N-Alkyl-N-Aralkylamino, N-Alkyl-N-Arylamino, Aminoalkyl, Alkylaminoalkyl, N-Phenylaminoalkyl, N-Phenylalkylaminoalkyl, N-Alkyl-N-Phenylalkylaminoalkyl, N-Alkyl-N-Phenylaminoalkyl, Phenyloxy, Phenylalkoxy, Phenylthio, Phenylalkylthio, Alkylsulfinyl, Alkylsulfonyl, Aminosulfonyl, Alkylaminosulfonyl, N-Phenylaminosulfonyl, Phenylsulfonyl und N-Alkyl-N-Phenylaminosulfonyl, ist; und
wobei R⁴ Hydrido oder Fluor ist;
oder einem pharmazeutisch annehmbaren Salz davon und wobei jeder Teil der geformten Zusammensetzung den selektiven Cyclooxygenase-2-Inhibitor in einer für ein nicht-menschliches Tier mit Körpergewicht größer als etwa 1 kg therapeutisch oder prophylaktisch wirksamen Menge enthält.

3. Herstellungsgegenstand nach Ansprüchen 1 bis 2, wobei der Cyclooxygenase-2-Inhibitor ausgewählt ist aus Celecoxib, Deracoxib, Rofecoxib und Valdecoxib.

4. Herstellungsgegenstand nach Ansprüchen 1 bis 2, wobei der Cyclooxygenase-2-Inhibitor Deracoxib ist.

5. Herstellungsgegenstand nach Ansprüchen 1 bis 2, wobei jeder Teil der geformten Zusammensetzung den selektiven Cyclooxygenase-2-Inhibitor in einer für ein nicht-menschliches Tier mit Körpergewicht größer als etwa 2 kg bis etwa 70 kg therapeutisch oder prophylaktisch wirksamen Menge enthält.

6. Herstellungsgegenstand nach Ansprüchen 1 bis 2, wobei jeder Teil der geformten Zusammensetzung den selektiven Cyclooxygenase-2-Inhibitor in einer für ein nicht-menschliches Tier mit Körpergewicht größer als etwa 50 kg bis etwa 1500 kg therapeutisch oder prophylaktisch wirksamen Menge enthält.

7. Herstellungsgegenstand nach Ansprüchen 1 bis 2, wobei jeder Teil der geformten Zusammensetzung den selektiven Cyclooxygenase-2-Inhibitor in einer für einen Hund therapeutisch oder prophylaktisch wirksamen Menge enthält.

8. Herstellungsgegenstand nach Ansprüchen 1 bis 2, wobei jeder Teil der geformten Zusammensetzung den selektiven Cyclooxygenase-2-Inhibitor in einer für ein Pferd therapeutisch oder prophylaktisch wirksamen Menge enthält.

9. Herstellungsgegenstand nach Ansprüchen 1 bis 2, wobei jeder Teil der geformten Zusammensetzung eine abgemessene Dosierungsmenge des selektiven Cyclooxygenase-2-Inhibitors zur Verabreichung an ein nicht-menschliches Tier enthält, die zwischen etwa 0,1 mg/kg Tierkörpergewicht bis etwa 15 mg/kg Tierkörpergewicht beträgt.

10. Herstellungsgegenstand nach Ansprüchen 1 bis 2, wobei jeder Teil der geformten Zusammensetzung eine abgemessene Dosierungsmenge des selektiven Cyclooxygenase-2-Inhibitors zur Verabreichung an ein nicht-menschliches Tier enthält, die zwischen etwa 0,5 mg/kg Tierkörpergewicht bis etwa 10 mg/kg Tierkörpergewicht beträgt.

## Revendications

1. Article de fabrication comprenant une composition profilée ayant deux extrémités sensiblement planaires, une dimension allongée sensiblement orthogonale aux extrémités et une zone transversale sensiblement uniforme, la composition profilée comprenant un composé alimentaire ayant un inhibiteur de la 2-cyclooxygénase sélectif distribué de manière homogène dans celle-ci, la composition profilée étant emballée dans un matériau d'emballage pouvant être coupé ayant des marques imprimées sur celui-ci à espacements égaux le long de la dimension allongée, lesdites marques correspondant à des augmentations de la quantité de dosage de l'inhibiteur de la 2-cyclooxygénase contenu dans des portions de la composition profilée définie par les marques,
dans lequel ledit inhibiteur de la 2-cyclooxygénase est choisi parmi une classe de composés répondant à la formule suivante : 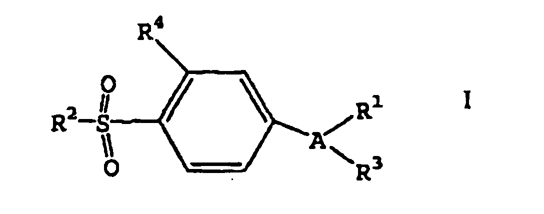 dans laquelle A est un substituant cyclique de 5 ou 6 éléments choisi parmi des cycles hetéréocyclo et carbocycliques instaurés ou partiellement insaturés ;
dans laquelle R¹ est un groupe cyclohexyle ou un groupe phényle éventuellement substitué par un, deux ou trois radicaux choisis parmi les groupes alkyle en C₁-C₂, halogénoalkyle en C₁-C₂, cyano, carboxy, (alcoxy en C₁-C₂)carbonyle, hydroxy, hydroxyalkyle en C₁-C₂, halogénoalcoxy en C₁-C₂, amino, aminoalkyle en C₁-C₂, aminophényle, nitro, (alcoxy en C₁-C₂) (alkyle en C₁-C₂), (alkyle en C₁-C₂) sulfinyle, halogène, alcoxy en C₁-C₂ et (alkyle en C₁-C₂) thio ;
dans laquelle R² est un groupe méthyle ou un groupe amino ;
dans laquelle R³ est un radical choisi parmi un atome d'halogène, des groupes alkyle en C₁-C₂, alcényle, alcynyle, oxo, cyano, carboxy, cyanoalkyle, hétérécyclyloxy, (alkyle en C₁-C₂)oxy, alkylthio, alkylcarbonyle, cycloalkyle, phényle, halogénoalkyle en C₁-C₂, hétérocyclo, cycloalcényle, phénylalkyle, hétérocyclylalkyle, alkylthioalkyle, hydroxyalkyle en C₁-C₂, alcoxycarbonyle, phénylcarbonyle, phénylalkylcarbonyle, phénylalcényle, alcoxyalkyle, phénylthioalkyle, phényloxyalkyle, phénylalkylthioalkyle, phénylalcoxyalkyle, alcoxyphénylalcoxyalkyle, alcoxycarbonylalkyle, aminocarbonyle, aminocarbonylalkyle, alkylaminocarbonyle, N-phénylaminocarbonyle, N-alkyl-N-phénylaminocarbonyle, alkylaminocarbonylalkyle, carboxyalkyle, alkylamino, N-arylamino, N-aralkylamino, N-alkyl-N-aralkylamino, N-alkyl-N-arylamino, aminoalkyle, alkylaminoalkyle, N-phénylaminoalkyle, N-phénylalkylaminoalkyle, N-alkyl-N-phénylalkylaminoalkyle, N-alkyl-N-phénylaminoalkyle, phényloxy, phénylalcoxy, phénylthio, phénylalkylthio, alkylsulfinyle, alkylsulfonyle, aminosulfonyle, alkylaminosulfonyle, N-phénylaminosulfonyle, phénylsulfonyle et N-alkyl-N-phénylaminosulfonyle ; et
dans laquelle R⁴ est un groupe hydrido ou un atome de fluor ;
ou un sel pharmaceutiquement acceptable de ceux-ci, et
dans lequel chaque augmentation de la quantité de dosage correspond à une portion de ladite composition profilée contenant ledit inhibiteur de la 2-cyclooxygénase sélectif en une quantité thérapeutiquement ou prophylactiquement efficace pour un animal non humain d'un poids corporel supérieur à environ 1 kg.

2. Article de fabrication comprenant une composition profilée qui comprend un produit alimentaire friable ayant un inhibiteur de la 2-cyclooxygénase sélectif distribué de manière sensiblement homogène dans celle-ci, la composition profilée ayant des moyens pour fournir des zones linéaires de résistance mécanique réduite permettant une rupture en des portions de taille sensiblement équivalentes, chacune contenant une quantité de dosage mesurée de l'inhibiteur de la 2-cyclooxygénase,
dans lequel ledit inhibiteur de la 2-cyclooxygénase est choisi parmi une classe de composés de la formule suivante : 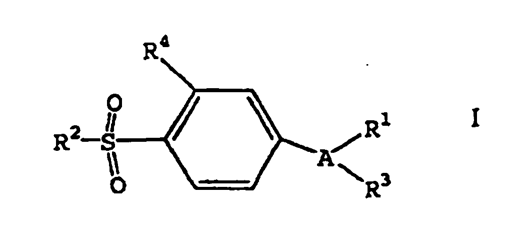 dans laquelle A est un substituant cyclique de 5 ou 6 éléments choisi parmi des cycles hetéréocylo et carbocycliques instaurés ou partiellement insaturés ;
dans laquelle R¹ est un groupe cyclohexyle ou un groupe phényle éventuellement substitué par un, deux ou trois radicaux choisis parmi les groupes alkyle en C₁-C₂, halogénoalkyle en C₁-C₂, cyano, carboxy, (alcoxy en C₁-C₂)carbonyle, hydroxy, hydroxyalkyle en C₁-C₂, halogénoalcoxy en C₁-C₂, amino, aminoalkyle en C₁-C₂, aminophényle, nitro, (alcoxy en C₁-C₂) (alkyle en C₁-C₂), (alkyle en C₁-C₂)sulfinyle, halogène, alcoxy en C₁-C₂ et (alkyle en C₁-C₂) thio ;
dans laquelle R² est un groupe méthyle ou un groupe amino ;
dans laquelle R³ est un radical choisi parmi un atome d'halogène, des groupes alkyle en C₁-C₂, alcényle, alcynyle, oxo, cyano, carboxy, cyanoalkyle, hétérécyclyloxy, (alkyle en C₁-C₂)oxy, alkylthio, alkylcarbonyle, cycloalkyle, phényle, halogénoalkyle en C₁-C₂, hétérocyclo, cycloalcényle, phénylalkyle, hétérocyclylalkyle, alkylthioalkyle, hydroxyalkyle en C₁-C₂, alcoxycarbonyle, phénylcarbonyle, phénylalkylcarbonyle, phénylalcényle, alcoxyalkyle, phénylthioalkyle, phényloxyalkyle, phénylalkylthioalkyle, phénylalcoxyalkyle, alcoxyphénylalcoxyalkyle, alcoxycarbonylalkyle, aminocarbonyle, aminocarbonylalkyle, alkylaminocarbonyle, N-phénylaminocarbonyle, N-alkyl-N-phénylaminocarbonyle, alkylaminocarbonylalkyle, carboxyalkyle, alkylamino, N-arylamino, N-aralkylamino, N-alkyl-N-aralkylamino, N-alkyl-N-alkylamino, aminoalkyle, alkylaminoalkyle, N-phénylaminoalkyle, N-phénylalkylaminoalkyle, N-alkyl-N-phénylalkylaminoalkyle, N-alkyl-N-phénylaminoalkyle, phényloxy, phénylalcoxy, phénylthio, phénylalkylthio, alkylsulfiniyle, alkylsulfonyle, alkylaminosulfonyle, N-phénylaminosulfonyle, phénylsulfonyle et N-alkyl-N-phénylaminosulfonyle ; et
dans laquelle R⁴ est un groupe hydrido ou un atome de fluor ;
ou un sel pharmaceutiquement acceptable de ceux-ci, et
dans lequel chaque augmentation de dosage correspond à une portion de ladite composition profilée contenant ledit inhibiteur de la 2-cyclooxygénase sélectif en une quantité thérapeutiquement ou prophylactiquement efficace pour un animal non humain d'un poids corporel supérieur à environ 1 kg.

3. Article de fabrication selon la revendication 1 ou 2, dans lequel ledit inhibiteur de la 2-cyclooxygénase est choisi parmi Celecoxib, Deracoxib, Rofecoxib et Valdecoxib.

4. Article de fabrication selon la revendication 1 ou 2, dans lequel ledit inhibiteur de la 2-cyclooxygénase est le Deracoxib.

5. Article de fabrication selon la revendication 1 ou 2, dans lequel chaque portion de ladite composition profilée contient ledit inhibiteur de la 2-cyclooxygénase en une quantité thérapeutiquement ou prophylactiquement efficace pour un animal non humain de poids corporel supérieur à environ 2 kg et allant jusqu'à environ 70 kg.

6. Article de fabrication selon la revendication 1 ou 2, dans lequel chaque portion de ladite composition profilée contient ledit inhibiteur de la 2-cyclooxygénase en une quantité thérapeutiquement ou prophylactiquement efficace pour un animal non humain de poids corporel supérieur à environ 50 kg et allant jusqu'à environ 1500 kg.

7. Article de fabrication selon la revendication 1 ou 2, dans lequel chaque portion de ladite composition profilée contient ledit inhibiteur de la 2-cyclooxygénase en une quantité thérapeutiquement ou prophylactiquement efficace pour un chien.

8. Article de fabrication selon la revendication 1 ou 2, dans lequel chaque portion de ladite composition profilée contient ledit inhibiteur de la 2-cyclooxygénase en une quantité thérapeutiquement ou prophylactiquement efficace pour un cheval.

9. Article de fabrication selon la revendication 1 ou 2, dans lequel chaque portion de ladite composition profilée contient une dose mesurée dudit inhibiteur de la 2-cyclooxygénase pour une administration à un animal non humain qui est d'environ 0,1 mg/kg de poids corporel de l'animal à environ 15 mg/kg de poids corporel de l'animal.

10. Article de fabrication selon la revendication 1 ou 2, dans lequel chaque portion de ladite composition profilée contient une dose mesurée dudit inhibiteur de la 2-cyclooxygénase pour une administration à un animal non humain qui est d'environ 0,5 mg/kg de poids corporel de l'animal à environ 10 mg/kg de poids corporel de l'animal.
